(19) Europäisches Patentamt
European Patent Office
Office européen des brevets

(11) Numéro de publication : **0 342 115 B1**

# (12) FASCICULE DE BREVET EUROPEEN

(45) Date de publication du fascicule du brevet : **09.09.92 Bulletin 92/37**

(51) Int. Cl.$^5$ : **C07C 317/08,** C07C 323/05

(21) Numéro de dépôt : **89401279.8**

(22) Date de dépôt : **09.05.89**

## (54) Nouveaux eicosanoides sulfurés et leur application en pharmacie et en cosmétique.

(30) Priorité : **10.05.88 FR 8806313**

(43) Date de publication de la demande : **15.11.89 Bulletin 89/46**

(45) Mention de la délivrance du brevet : **09.09.92 Bulletin 92/37**

(84) Etats contractants désignés : **AT BE CH DE FR GB IT LI NL SE**

(56) Documents cités : **Eléments de la technique relevés: néant.**

(73) Titulaire : **CENTRE INTERNATIONAL DE RECHERCHES DERMATOLOGIQUES GALDERMA - CIRD GALDERMA Sophia Antipolis F-06560 Valbonne (FR)**

(72) Inventeur : **Shroot, Braham Villa 35 Hameaux de Val Bosquet F-06600 Antibes (FR)**
Inventeur : **Hensby, Christopher 89, Chemin d'Andon Villa Madru F-06410 Biot (FR)**
Inventeur : **Maignan, Jean 8, rue Halévy F-93290 Tremblay-les-Gonesse (FR)**
Inventeur : **Lang, Gérard 44, avenue Lacour F-Saint-Gratien (FR)**
Inventeur : **Colin, Michel 1 Allée Cécile F-93190 Livry-Gargan (FR)**

(74) Mandataire : **Casalonga, Axel et al BUREAU D.A. CASALONGA - JOSSE Morassistrasse 8 W-8000 München 5 (DE)**

EP 0 342 115 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux dérivés d'acides eicosatriynoïque-5,8,11 et eicosatétraynoïque-5,8,11,14 et à leur application, d'une part, comme agents thérapeutiques dans le traitement ou la prophylaxie des maladies allergiques, dans le traitement des dermatoses et des maladies inflammatoires, et d'autre part, dans des compositions cosmétiques.

Il est connu qu'un certain nombre de substances jouent un rôle important dans le processus inflammatoire de la peau telles que l'acné, les dermatoses, comme par exemple le psoriasis, l'eczéma, etc... Ces substances, parmi lesquelles les prostaglandines, les acides hydroxyeicosatétraénoïques, les thromboxanes et les leucotriènes, ont toutes une origine commune qui est l'acide arachidonique (voir en particulier "VOORHEES-Leukotrienes and Other Lipoxygenase Products in the Pathogenesis and Therapy of Psoriasis and Other Dermatoses" Arch. Dermatol., Vol. II9, Juillet I983, 54I-547).

La formation de ces substances résulte essentiellement de la transformation après libération de l'acide arachidonique lié par une liaison ester aux lipides présents dans l'épiderme (par exemple les phospholipides).

On a déjà préconisé antérieurement, pour le traitement des maladies de la peau, soit des inhibiteurs de la cyclooxygénase empêchant la formation des prostaglandines tels que l'indométhacine, la vitamine E, etc... ; ou alors des substances susceptibles d'inhiber les lipoxygénases tels que l'acide eicosatétraynoïque.

On a également proposé pour le traitement du psoriasis l'acide eicosatétraynoïque-5,8,II,I4 ainsi que l'acide eicosatriynoïque-5,8,II et leurs esters d'alkyle inférieurs, notamment dans le brevet US-A-4 I90 669.

La demanderesse a découvert que, de façon surprenante, en remplaçant le groupement méthylène en position-3 dans la structure de l'acide eicosatriynoïque-5,8,II ou de l'acide eicosatétraynoïque-5,8,II,I4, par un hétéroatome tel que le soufre ou par un groupement sulfoxyde ou sulfone, on obtenait des produits qui inhibaient le métabolisme enzymatique de l'acide arachidonique provoqué par la cyclooxygénase et les lipoxygénases.

Ces acides ayant une fonction thioéther en position 3 présentent en outre l'avantage d'être d'un prix de revient beaucoup plus intéressant puisque les deux dernières étapes de synthèse font intervenir l'alcool propargylique et l'acide thioglycolique qui sont deux matières premières très accessibles.

Les composés thioéther, sulfoxyde ou sulfone présentent, de façon surprenante, une biodisponibilité différente de celle des acides correspondants eicosatriynoïque-5,8,II ou eicosatétraynoïque-5,8,II,I4.

En effet, avec cette nouvelle fonction sulfoxyde et surtout sulfone, on peut obtenir des produits de polarité très différente. Par exemple, lorsque c'est une fonction sulfone, les acides correspondants dioxo-3,3 thia-3 eicosatriynoïque-5,8,II et dioxo-3,3 thia-3 eicosatétraynoïque-5,8,II,I4 salifiés par une amine tertiaire, telle que la triéthanolamine, sont solubles dans l'eau à plus de IO%, alors que la solubilité des sels des acides dont la chaîne est entièrement carbonée, est inférieure à I%.

On sait, par ailleurs, que les esters d'alkyle inférieur de ces deux acides carbonés sont instables et se dégradent au cours leur purification, alors que, par exemple, le dioxa-3,3 thia-3 eicosatriynoate-5,8,II de méthyle est isolé sans problème.

L'invention a donc pour objet ces nouveaux acides ainsi que leurs dérivés tels que les esters et amides.

Un objet de l'invention est également constitué par le procédé de préparation de ces dérivés.

Un autre objet de l'invention est constitué par les compositions pharmaceutiques contenant, à titre de substance active, de tels composés.

L'invention a enfin pour objet l'utilisation, dans le domaine de la cosmétique, de ces composés et les compositions cosmétiques correspondantes, notamment dans des compositions antiacnéiques, solaires ou post-solaires ou dans le traitement des dermatites séborrhéiques.

D'autres objets de l'invention apparaîtront à la lecture de la description et des exemples qui suivent.

Les composés conformes à l'invention sont essentiellement caractérisés par le fait qu'ils répondent à la formule générale (I) :

$$C_5H_{11}-A-CH_2-C\equiv C-CH_2-C\equiv C-CH_2-C\equiv C-CH_2-\underset{\underset{(O)_n}{\downarrow}}{S}-CH_2-COR \qquad (I)$$

dans laquelle :

A correspond aux radicaux suivants :

$-(CH_2)_2-$ ou $-(C\equiv C)-$;

n est égal à 0,1 ou 2;

R représente un radical hydroxyle, alcoxy de formule $-OR_1$, ou un radical amino de formule :

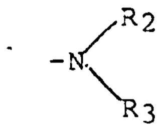

dans lesquelles :

$R_1$ représente un radical alkyle ayant de 1 à 20 atomes de carbone, monohydroxyalkyle, polyhydroxyalkyle, aryle ou aralkyle éventuellement substitué(s) ou un reste de sucre;

$R_2$ et $R_3$ représentent indépendamment l'un de l'autre, un atome d'hydrogène, un radical alkyle inférieur, un radical monohydroxyalkyle éventuellement interrompu par un hétéroatome, un radical polyhydroxyalkyle, un radical aryle ou benzyle éventuellement substitué(s), un reste d'amino acide ou de sucre aminé;

$R_2$ et $R_3$ peuvent également former avec l'atome d'azote auquel ils sont rattachés, un hétérocycle comportant éventuellement un hétéroatome supplémentaire et/ou étant éventuellement substitué par un groupement alkyle ou hydroxyalkyle,

ainsi que leurs sels et les isomères optiques et géométriques.

On entend par radical alkyle inférieur, un radical ayant 1 à 6 atomes de carbone.

Parmi les radicaux alkyle ayant jusqu'à 20 atomes de carbone, on peut citer les radicaux méthyle, éthyle, isopropyle, butyle, tertiobutyle, éthyl-2 hexyle, isooctyle, dodécylé, hexadécyle et octadécyle.

On entend par radical monohydroxyalkyle, éventuellement interrompu par un hétéroatome, un radical ayant de 2 à 6 atomes de carbone, éventuellement interrompu par un atome d'oxygène et plus particulièrement un radical hydroxy-2 éthyle, hydroxy-2 propyle ou hydroxy-2 éthoxyéthyle.

Par radical polyhydroxyalkyle, on entend de préférence un radical contenant de 3 à 6 atomes de carbone et de 2 à 5 groupes hydroxyle, tels que les radicaux dihydroxy-2,3 propyle, dihydroxy-l,3 propyl-2 ou le reste du pentaérythritol.

Par radical aryle, on entend un radical phényle éventuellement substitué par au moins un atome d'halogène, un groupement -OH ou $-NO_2$, un radical alkyle inférieur, un radical trifluorométhyle ou une fonction acide carboxylique.

Les radicaux aralkyle préférés sont les radicaux benzyle ou phénétyle.

Par reste d'un sucre, on entend de préférence un radical dérivant du glucose, du mannose, de l'érythrose ou du galactose.

Des restes de sucres aminés sont en particulier ceux dérivant de la glucosamine, de la galactosamine, de la mannosamine ou de la méglumine.

Lorsque les radicaux $R_2$ et $R_3$, pris ensemble, forment avec l'atome d'azote auquel ils sont rattachés, un hétérocycle, celui-ci est de préférence un radical pipéridino, pipérazino, morpholino, pyrrolidino ou (hydroxy-2 éthyl)-4 pipérazino.

Les sels des composés conformes à l'invention sont en particulier choisis parmi les sels de métal alcalin ou alcalino-terreux, ou encore parmi les sels de zinc, d'une amine organique ou les sels d'ammonium quaternaires, lorsqu'ils comportent au moins une fonction acide libre; soit parmi les sels d'un acide minéral ou organique, notamment les chlorhydrate, bromhydrate ou citrate, lorsqu'ils comportent au moins une fonction amine.

Les acides particulièrement préférés, conformes à l'invention, sont les acides :

- thia-3 eicosatriynoïque-5,8,ll;
- thia-3 eicosatétraynoïque-5,8,ll,l4;
- dioxo-3,3 thia-3 eicosatriynoïque-5,8,ll;
- dioxo-3,3 thia-3 eicosatétraynoïque-5,8,ll,l4;

et les amides de ceux-ci.

Les composés conformes à l'invention peuvent être préparés par mise en oeuvre des procédés suivants :

L'acide thia-3 eicosatriynoïque-5,8,ll est préparé suivant le schéma réactionnel A à partir du décyne-l (l). L'anion de ce dernier, formé par réaction avec une base forte tel qu'un halogène magnésien d'alkyle, est mis en réaction avec un excès de dihalogéno-l,4 butyne-2 et conduit à l'halogéno-l tétradécadiyne-2,5 (2) dont

la préparation est décrite dans le brevet français 2 584 400.

Cet halogénure (2) est mis en réaction avec le dianion de l'alcool propargylique formé au préalable en traitant cet alcool avec 2 équivalents basiques. Les bases utilisées sont des bases fortes telles que les organolithiens comme par exemple le butyl-lithium ou des organomagnésiens tels que l'halogéno magnésien d'éthyle ou de propyle dans un solvant anhydre, de préférence un éther comme le tétrahydrofuranne ou le diéthyléther.

On obtient après acidification du milieu réactionnel, l'heptadécatriyne-2,5,8 ol-l (3) que l'on purifie par recristallisation.

Dans une troisième étape, cet alcool (3) est traité dans un solvant chloré tel que le dichlorométhane ou le dichloro-l,2 éthane ou un éther par un trihalogénure de phosphore.

L'halogéno-l heptadécatriyne-2,5,8 (4) est alors mis en réaction directement avec le dianion de l'acide thioglycolique : $HS-CH_2-CO_2H$ formé en traitant ce dernier avec 2 équivalents de base.

Dans ce dernier cas, les bases employées sont minérales ou organiques, les bases préférées étant cependant la soude, la potasse ou le méthanolate de sodium.

L'acide thia-3 eicosatriynoïque -5,8,ll, (5) est purifié par cristallisation dans un solvant approprié.

## SCHEMA REACTIONNEL A

$C_8H_{17}-C{\equiv}C-H$

(1)

1) $B^-$

2) $X-CH_2-C{\equiv}C-CH_2X$

$C_8H_{17}-C{\equiv}C-CH_2-C{\equiv}C-CH_2X$

(2)

1) $H-C{\equiv}C-CH_2-OH$, $2B^-$

2) $H^+$

$C_8H_{17}-C{\equiv}C-CH_2-C{\equiv}C-CH_2-C{\equiv}C-CH_2OH$

(3)

$PX_3$

$C_8H_{17}-C{\equiv}C-CH_2-C{\equiv}C-CH_2-C{\equiv}C-CH_2X$

(4)

1) $HSCH_2CO_2H$, $2B^-$

2) $H^+$

$C_8H_{17}-C{\equiv}C-CH_2-C{\equiv}C-CH_2-C{\equiv}C-CH_2SCH_2CO_2H$

(5) [(Composé de formule générale (I) dans laquelle A = $(CH_2)_2$]

Les acides tétraynoïques sont synthétisés suivant le même principe, en mettant en oeuvre le schéma réactionnel B et en partant de l'heptyne-1 (6).

L'anion de ce dernier traité par le dihalogéno-1,4 butyne conduit à l'halogéno-1 undécadiyne-2,5 (7). Cette synthèse est décrite plus particulièrement dans la demande de brevet français n° 86 18 419.

Le passage de l'halogénure ayant 11 atomes de carbone de formule (7) à l'hydroxy-1 tétradécatriyne-2,5,8 (8) est réalisé par action du dianion de l'alcool propargylique. Cet alcool de formule (8) est à son tour transformé en halogéno-1 tétradécatriyne-2,5,8 de formule (9) par action d'un trihalogénure de phosphore.

Suivant les conditions expérimentales identiques aux deux étapes précédentes, l'halogénure (9) est transformé par action du dianion de l'alcool propargylique en heptadécatétrayne-2,5,8,11 ol-1 (10). Ce dernier est transformé en halogéno-1 heptadécatétrayne-2,5,8,11 (11) par action du trihalogénure de phosphore.

La synthèse du bromo-1 heptadécatétrayne-2,5,8,11 est décrite dans le brevet US-A-3 033 884.

Comme dans le cas du schéma réactionnel A, l'halogéno-1 heptadécatétrayne-2,5,8,11 (11) est alors mis en réaction directement avec le dianion de l'acide $H-S-CH_2CO_2H$ formé en traitant ce dernier avec 2 équivalents de base.

SCHEMA REACTIONNEL B

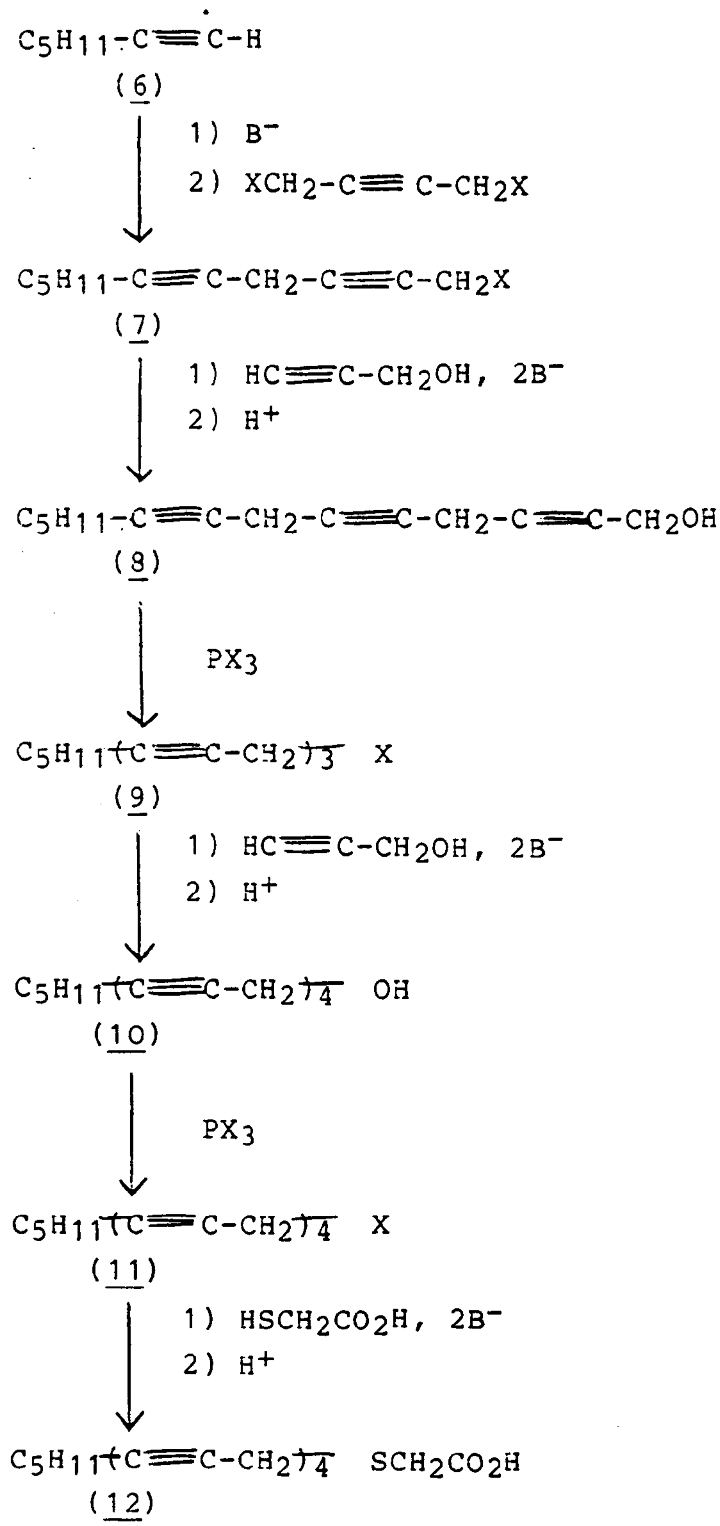


Les acides de formule générale (I') peuvent être transformés en esters (II) correspondants suivant les méthodes habituelles de transformation d'un acide en ester, c'est-à-dire par action d'un alcool en milieu acide ou par réaction de déplacement de l'halogène d'un halogénure d'alkyle par la fonction carboxylate de sodium ou de potassium de l'acide (I').

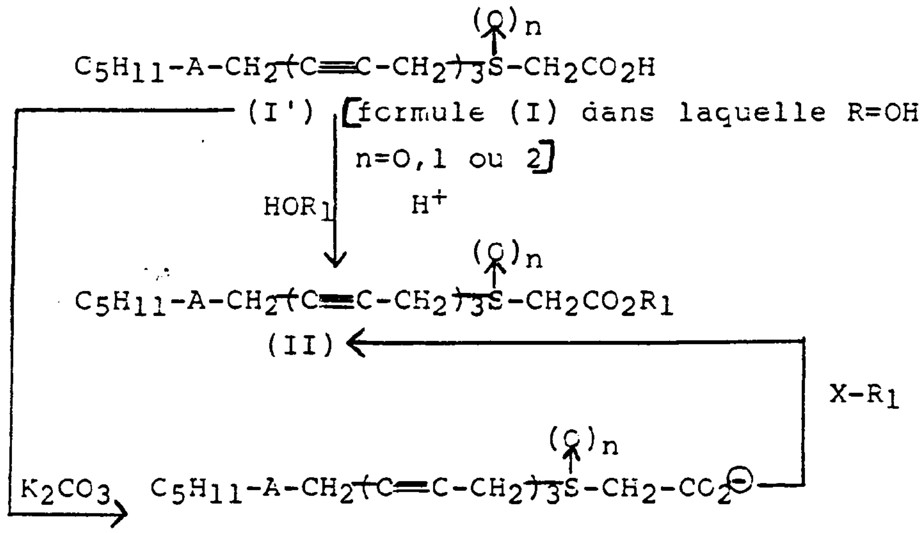


Dans la formule précitée, $R_1$ a les significations indiquées ci-dessus et X désigne un atome d'halogène tel que chlore ou brome.

Les amides de formule générale (III) rentrant dans la définition de la formule générale (I) dans laquelle R désigne le groupement :

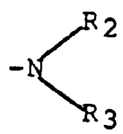

conforme à l'invention, sont obtenus en faisant réagir une forme activée des acides de formule (I') sur une amine dans un solvant organique. Cette forme activée de l'acide peut être, soit un chlorure d'acide, soit un anhydride ou encore l'intermédiaire formé par addition, à une solution d'acide, de carbonyl diimidazole (C.D.I.).

Cette dernière réaction est conduite de préférence en milieu solvant tel que le diméthylformamide ou encore un solvant chloré comme le dichlorométhane ou le dichloro-l,2 éthane. Cette réaction se déroule en particulier selon le schéma réactionnel suivant :

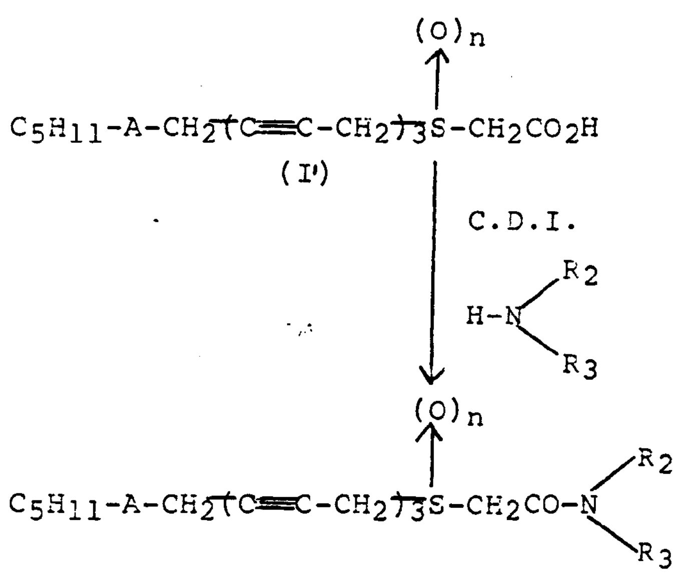


(III)

Lorsque les thioglycolamides sont facilement accessibles, les amides de structure (III) peuvent être obtenus directement sans passer par l'intermédiaire de l'acide de formule (I') (Schéma réactionnel C), en traitant l'halogénure (4) ou (II) par le thiolate formé au préalable à partir du thioglycolamide (I3). Ce dernier est préparé par action d'une amine

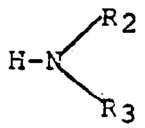

sur le thioglycolate d'éthyle (I4). Cette méthode est en effet plus simple. On prépare les halogénures (4) ou (II), d'une part, et le sel de sodium ou de potassium du thioglycolamide, d'autre part, dans le méthanol ou l'éthanol. Les halogénures (4) ou (11) ne sont pas purifiés et leur mélange réactionnel est directement ajouté à une solution alcoolique du thioglycolamide salifié par un équivalent de base.

SCHEMA REACTIONNEL C

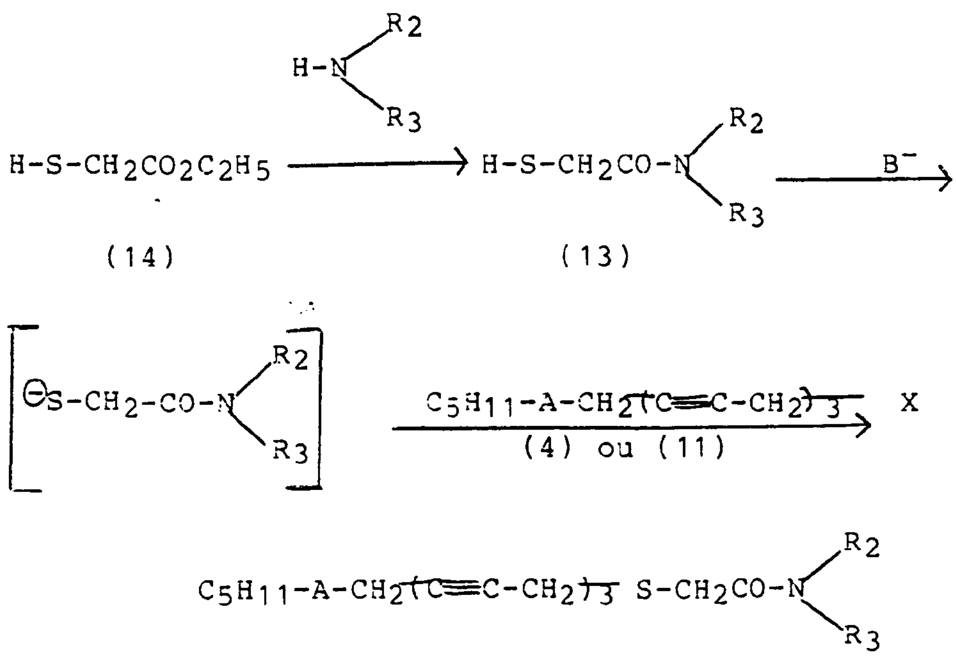


(14)

(13)

(I) [où A=$(CH_2)_2$ ou -C≡C-]

Les sulfoxydes ou les sulfones de formule générale (I) (n=1 ou 2) sont préparés à partir des thioéthers correspondants par action d'un équivalent ou de deux équivalents d'un peracide organique (15) dans un solvant organique tel que le chlorure de méthylène ou le dichloro-1,2 éthane. (Schéma réactionnel D)

SCHEMA REACTIONNEL D

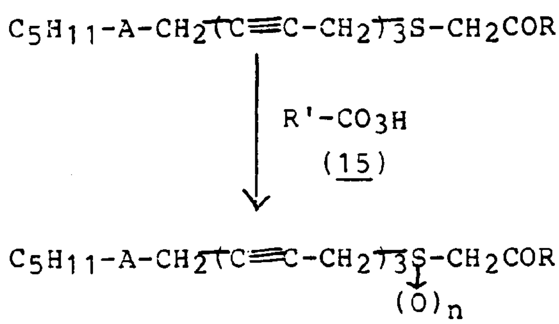


1 équivalent de (15) n=1

2 équivalents de (15) n=2

Les sulfoxydes entrant dans la définition de la formule générale (I) peuvent également être synthétisés par oxydation du thioéther correspondant, au bromite de sodium.

Les composés de formule (I), conformes à l'invention, ont une activité particulièrement remarquable vis-à-vis de l'inhibition du métabolisme de l'acide arachidonique et notamment au niveau des lipoxygénases à l'origine de la formation des leucotriènes et des acides hydroxylés qui jouent un rôle important dans le processus inflammatoire.

Ils peuvent être administrés à l'homme ou à l'animal à l'aide de compositions contenant en plus un support ou diluant pharmaceutiquement acceptable. Ces composés peuvent également être associés, si on le souhaite, à d'autres substances actives n'ayant pas d'effet antagoniste.

Les compositions pharmaceutiques ou vétérinaires conformes à l'invention peuvent être administrées par voie systémique ou locale.

Pour la voie entérale, elles peuvent se présenter sous forme de comprimés, de gélules, de dragées, de suppositoires, de sirops, de suspensions, de dispersions, de solutions, de poudres, de granulés, d'émulsions, etc.

Pour la voie topique, les compositions pharmaceutiques se présentent, entre autres, sous forme d'onguents, de teintures, de crèmes, de pommades, de poudres, de timbres, de tampons imbibés, de solutions, de lotions, de gels, de sprays, de shampooings, de suspensions, ou encore de microdispersions de vésicules lipidiques ioniques ou nonioniques renfermant facultativement une huile.

Les compositions utilisées par voie topique peuvent se présenter, soit sous forme anhydre, soit sous forme aqueuse, selon l'indication clinique.

Pour la voie ophtalmique, on peut utiliser un collyre aqueux stérile sous la forme d'une suspension ayant une taille de particules inférieure à 25 um selon les indications pharmacopée. On peut également proposer une pommade ophtalmique où la substance active serait solubilisée. Cette pommade serait constituée d'un mélange approprié de polyéthylèneglycol.

Pour les voies aériennes, on utilise des pulvérisations buccopharyngées de solutions hydroéthanoliques, ou de suspensions dans une phase dispersive avec une taille de particules appropriée.

Les compositions pharmaceutiques conformes à l'invention peuvent également être administrées par voie parentérale et notamment par voie intraveineuse, intramusculaire, intrapéritonéale, sous-cutanée ou intradermique.

Pour les voies parentérales, et plus particulièrement les injections, on utilise la substance active dans un véhicule stérile tel que l'eau. La substance active est soit mise en suspension ou dissoute dans ce véhicule ou dans des vésicules lipidiques ioniques ou non ioniques.

Pour la voie intramusculaire ou sous-cutanée, les compositions peuvent se présenter sous la forme :

– d'une suspension dans un soluté aqueux dispersif, par exemple un mélange d'alcool benzylique, de carboxyméthylcellulose sodique, de chlorure de sodium, du produit de condensation de 20 moles d'oxyde

d'éthylène sur un mélange d'esters oléiques du sorbitol et d'anhydrides du sorbitol dénommé"Polysorbate 80", d'eau pour préparation injectable;

– d'une solution dans un solvant non aqueux constitué d'une huile végétale pour préparations injectables (ex. huile de ricin) additionnée ou non d'un faible pourcentage d'alcool éthylique. La stérilisation du soluté non aqueux sera faite par filtration.

Les composés, conformes à l'invention, peuvent également être utilisés en cosmétique, notamment dans les crèmes, les lotions pour la peau telles que dans les produits solaires, post-solaires, apaisants, antiséborrhéiques, antiacnéiques ou encore les shampooings.

Les compositions pharmaceutiques et/ou cosmétiques, conformes à l'invention, peuvent contenir des additifs inertes ou pharmacodynamiquement ou cosmétiquement actifs, et notamment :

– des agents hydratants comme la thiamorpholinone et ses dérivés ou l'urée; des agents antiséborrhéiques tels que la S-carboxyméthylcystéine, la S-benzylcystéamine et leurs dérivés, la tioxolone; des antibiotiques comme l'érythromycine et ses esters, la néomycine, la clindamycine et ses dérivés, ou les tétracyclines; des agents qui interfèrent avec la kératinisation tels que l'acide salicylique et les pipéridino-6 pyrimidine oxyde-3) et ses dérivés, le diazoxyde et le Phénytoïn; d'autres agents anti-inflammatoires stéroïdiens ou non stéroïdiens; des caroténoïdes et notamment le β-carotène; des agents anti-psoriasiques tels que l'anthraline et ses dérivés; des inhibiteurs de phospholipases $A_2$; des antifongiques tels que les imidazoles, les triazoles, les allylamines ou les thiocarbamates.

Les compositions conformes à l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des antioxydants tels que l'α-tocophérol, le butylhydroxyanisole ou le butylhydroxytoluène, les anesthésiques locaux, des tampons, etc...

Les compositions conformes à l'invention peuvent être conditionnées sous des formes retard ou à libération progressive, connues en elles-mêmes. Elles peuvent enfin contenir des vésicules ioniques (liposomes) ou non ioniques.

La substance active, répondant à la formule générale (I), conforme à l'invention, est présente dans les compositions pharmaceutiques ou cosmétiques, dans des proportions comprises entre 0,01 et 10% en poids par rapport au poids total de la composition et de préférence entre 0,1 et 5% en poids.

Dans l'application thérapeutique, le traitement est déterminé par le médecin et peut varier suivant l'âge, le poids et la réponse du patient ainsi que la gravité des symptômes. Le dosage est généralement compris entre 0,05 et 500 mg/kg/jour et de préférence entre 0,5 et 100 mg/kg/jour. La durée du traitement est variable suivant la gravité des symptômes et peut s'étaler entre 1 et 12 semaines de façon continue ou discontinue.

L'invention a également pour objet l'utilisation des composés de formule (I) dans la préparation de compositions pharmaceutiques ou vétérinaires destinées au traitement ou à la prophylaxie des maladies allergiques et dans le traitement des dermatoses et des maladies inflammatoires.

Les exemples suivants sont destinés à illustrer l'invention sans pour autant présenter un caractère limitatif.

EXEMPLE DE REFERENCE A

Préparation de l'heptadécatriyne-2,5,8 ol-1 (3)

Dans un premier stade, on prépare le chlorure de propyl magnésium en ajoutant goutte à goutte une solution de 53 g de chlorure de propyle dilué dans 100 $cm^3$ de THF (tétrahydrofuranne) anhydre, à une suspension agitée, à la température ordinaire sous atmosphère inerte, de 16,43 g de magnésium dans 300 $cm^3$ de THF anhydre. La réaction est terminée en portant le mélange réactionnel au reflux du THF pendant trois heures, après la fin de l'introduction du chlorure de propyle.

Dans un deuxième stade, on forme le dianion de l'alcool propargylique en ajoutant sous agitation, au mélange réactionnel précédent refroidi à 0°C, 19 g d'alcool propargylique. A la fin de l'introduction, le mélange est encore agité pendant 1 h 30 à la température ordinaire. Puis on ajoute 0,5 g de cyanure de cuivre et toujours à la température ordinaire, on agite encore pendant 1 h 30. Puis au mélange ainsi obtenu, refroidi à 0°C, on introduit goutte à goutte 40,54 g de chloro-1 tétradécadiyne-2,5. On abandonne le mélange une nuit à la température ordinaire et ensuite il est porté sous reflux pendant 9 heures. Le solvant est alors éliminé par évaporation sous vide et le résidu obtenu extrait au chlorure de méthylène. La phase organique est ensuite lavée à l'eau, séchée sur sulfate de magnésium et concentrée sous pression réduite. Le solide obtenu est solubilisé dans 350 $cm^3$ d'hexane bouillant. Par refroidissement, l'alcool attendu cristallise. Il est essoré et séché. On obtient 25 g d'heptadécatriyne-2,5,8 ol-1 sous forme de cristaux blancs dont le point de fusion est de 48°C. Son spectre de $^1H$ RMN est conforme à la structure attendue.

EXEMPLE DE REFERENCE B

Préparation de l'heptadécatétrayne-2,5,11 ol-1

Comme dans l'exemple A, on prépare le dianion de l'alcool propargylique par échange avec le chlorure de propylmagnésium.

Dans un premier temps, on traite 18 g de magnésium, dans 600 $cm^3$ de THF anhydre, par 72 $cm^3$ de chloropropane. Ensuite, lorsque le magnésium est totalement transformé, on ajoute alors, à la température ordinaire, 21,8 $cm^3$ d'alcool propargylique. Après 2 heures d'agitation à la température ordinaire, on introduit 3,5 g de cyanure cuivreux.

Le mélange est encore agité 2 heures à température ordinaire, puis refroidi à 0°C, température à laquelle est ajouté un équivalent de bromo-1 tétradécatriyne-2,5,8, préparé comme suit.

A une solution de 50 g de tétradécatriyne-2,5,8 ol-1 dans 300 $cm^3$ d'éther anhydre, on ajoute 12,5 $cm^3$ de tribromure de phosphore, puis 0,1 $cm^3$ de pyridine. Le mélange ainsi obtenu est porté au reflux pendant 3 heures. Ensuite, à la température ordinaire, il est versé sur une solution de bicarbonate de sodium. La phase éthérée est décantée, lavée à l'eau, séchée sur sulfate de magnésium, puis évaporée sous pression réduite. Le bromo-1 tétradécatriyne-2,5,8 est solubilisé dans 30 $cm^3$ de THF anhydre et introduit dans le mélange réactionnel contenant le dianion de l'alcool propargylique.

A la fin de l'introduction, le mélange est agité 2 heures puis abandonné une nuit. Il est alors versé sur une solution glacée de chlorure d'ammonium, puis extrait deux fois à l'acétate d'éthyle. Les phases d'acétate d'éthyle rassemblées sont lavées à l'eau, séchées sur sulfate de magnésium. Le solvant est alors éliminé par évaporation sous vide. Le produit noirâtre obtenu est extrait cinq fois à l'aide de 150 $cm^3$ d'heptane bouillant. Par refroidissement, l'heptadécatétrayne-2,5,8,11 ol-1 cristallise. On obtient 25 g de cristaux blanc-crème dont le point de fusion est de 54-56°C.

EXEMPLE DE PREPARATION I

Préparation de l'acide thia-3 eicosatriynoïque-5,8,11

On prépare séparément le bromo-1 heptadécatriyne-2,5,8.

Un mélange agité de 15 g d'heptadécatriyne-2,5,8 ol-1 (3), de 2,2 $cm^3$ de tribromure de phosphore, de 3 gouttes de pyridine dans 150 $cm^3$ d'éther anhydre est porté sous atmosphère inerte à ébullition pendant 2 heures. Puis, à température ordinaire, le mélange est lavé au bicarbonate de sodium puis à l'eau et séché sur sulfate de magnésium. Cette solution filtrée est ajoutée directement à une solution de thioglycolate de potassium préparée comme suit.

A la température ordinaire, sous atmosphère inerte, on ajoute 8,9 g de potasse à 4,7 $cm^3$ d'acide thioglycolique solubilisé dans 150 $cm^3$ de méthanol. L'agitation est maintenue pendant 2 heures à la température ordinaire. Au dianion de l'acide thioglycolique ainsi formé, on ajoute alors la solution éthérée de bromure préparée précédemment. La réaction de déplacement du bromure est très rapide. Lorsque ce dernier est totalement transformé, le mélange réactionnel est versé dans l'eau acidulée. La phase éthérée est décantée, lavée à l'eau, séchée sur sulfate de magnésium et le solvant évaporé. L'acide brut obtenu est recristallisé deux fois dans l'heptane bouillant. On obtient 9 g d'acide thia-3 eicosatriynoïque-5,8,11 sous forme de cristaux beiges fondant à 63°C.

Les spectres $^1H$ et $^{13}C$ RMN sont conformes à la structure attendue.

Le produit attendu est analysé sous forme de demi-hydrate.

Analyse élémentaire : $C_{19}H_{26}O_2S$, 1/2 $H_2O$

| | C | H | O | S |
|---|---|---|---|---|
| Calculé | 69,68 | 8,31 | 12,21 | 9,79 |
| Trouvé | 69,75 | 7,52 | 12,77 | 9,93 |

EXEMPLE DE PREPARATION 2

Préparation du N-(éthyl)thia-3 eicosatriynamide-5,8,11

a) Préparation du N-éthylthioglycolamide

Un mélange de 60 g de thioglycolate d'éthyle et de 150 $cm^3$ d'une solution aqueuse à 33% d'éthylamine est agité pendant 48 heures sous atmosphère inerte. Le N-éthylthioglycolamide est purifié par distillation sous vide. On obtient 47 g d'un liquide incolore de point d'ébullition 95-98°C/1,3 x $10^2$ Pa.

b) Préparation du thiolate de sodium du N-éthylthioglycolamide

On agite, pendant une heure, 2,43 g de N-éthylthioglycolamide en solution dans 30 $cm^3$ de méthanol avec 1,2 g de méthanolate de sodium à 0°C sous atmosphère inerte.

c) Préparation du bromo-1 heptadécatriyne-2,5,8

Un mélange de 5 g d'heptadécatriyne-2,5,8 ol-1, d'une goutte de pyridine et de 2 g de tribromure de phosphore dans 20 $cm^3$ d'éther éthylique est portée sous reflux pendant 3 heures. La phase éthérée est ensuite lavée au bicarbonate de sodium, puis à l'eau et séchée sur sulfate de magnésium.

d) Condensation du N-éthylthioglycolamide sur le bromo-1 heptadécatriyne-2,5,8

La solution éthérée de bromo-1 heptadécatriyne -2,5,8 préparée suivant le mode opératoire ci-dessus est ajoutée goutte à goutte à une solution agitée à 0°C sous atmosphère inerte, de thiolate de sodium du N-éthylthioglycolamide préparée suivant le mode opératoire b) ci-dessus. Le mélange obtenu est agité pendant l heure après la fin de l'addition. Les solvants sont ensuite éliminés par évaporation sous vide. Le produit obtenu est dissous dans 100 $cm^3$ de chlorure de méthylène. La phase organique est lavée à l'eau, séchée sur sulfate de magnésium, concentrée, puis déposée sur une colonne de gel de silice. L'amide attendu est élué au mélange chlorure de méthylène-acétate d'éthyle (97-3). Après évaporation de l'éluant, on isole 6 g de produit qui sont recristallisés dans l'éther isopropylique. On obtient 4 g de N-(éthyl) thia-3 eicosatriynamide-5,8,11. Ce sont des cristaux beiges de point de fusion de 59°C.
Analyse élémentaire : $C_{21}H_{31}N OS$

| | C | H | N | O | S |
|---|---|---|---|---|---|
| Calculé | 72,99 | 9,04 | 4,05 | 4,63 | 9,29 |
| Trouvé | 73,18 | 9,08 | 3,85 | 4,83 | 9,32 |

EXEMPLE DE PREPARATION 3

Préparation du N-(hydroxy-2 éthyl)thia-3 eicosatriynamide-5,8,11

a) Préparation du N-(hydroxy-2 éthyl)thioglycolamide

Un mélange agité sous atmosphère inerte de 13 g de thioglycolate d'éthyle et de 17 g d'éthanolamine est porté pendant 1 heure à une température de 70-80°C, puis une demi-heure à 130°C.
Le N-(hydroxy-2 éthyl)thioglycolamide est purifié par distillation Teb = 152°C/5,3 Pa.

b) Préparation du thiolate de sodium d'(hydroxy-2 éthyl)thioglycolamide

A une solution agitée à 0°C sous atmosphère inerte, de 1,35 g de N-(hydroxy-2 éthyl)thioglycolamide dans 15 $cm^3$ de méthanol, on ajoute 0,54 g de méthanolate de sodium solubilisé dans 15 $cm^3$ de méthanol.
L'agitation est maintenue 1 heure avant l'introduction du dérivé bromé.

c) Préparation du bromo-1 heptadécatriyne-2,5,8

Ce bromure est préparé comme dans l'exemple 2c) à partir de 2,44 g d'heptadécatriyne-2,5,8 ol-1 que l'on traite par 1 g de tribromure de phosphore dans 15 $cm^3$ d'éther éthylique.

d) La solution éthérée de bromo-1 heptadécatriyne-2,5,8 est ajoutée à la solution méthanolique de thiolate de sodium du N-(hydroxy-2 éthyl)thioglycolamide. A la fin de la réaction, le mélange de solvants est éliminé par évaporation sous vide. Le produit est solubilisé dans 50 $cm^3$ de chlorure de méthylène.

La phase organique est lavée à l'eau, décantée, séchée sur sulfage de magnésium, concentrée et déposée sur une colonne de chromatographie de gel de silice. En utilisant comme éluant le chlorure de méthylène, on élimine les impuretés et ensuite à l'acétate d'éthyle, l'amide est entraîné. Après évaporation de l'éluant, les cristaux obtenus sont lavés au pentane. On isole ainsi 1,5 g de N-(hydroxy-2 éthyl) thia-3 eicosatriynamide-5,8,11 dont le point de fusion est de 77°C. Les spectres IR et $^1H$ RMN sont conformes à la structure attendue.

L'analyse élémentaire correspond à un produit partiellement hydraté : $C_{21}H_{31}NO_2S$, 1/4 $H_2O$

| | C | H | N | O | S |
|---|---|---|---|---|---|
| Calculé | 68,90 | 8,67 | 3,82 | 9,83 | 8,75 |
| Trouvé | 68,78 | 8,75 | 3,85 | 10,25 | 8,75 |

EXEMPLE DE PREPARATION 4

Préparation du N-(éthyl)oxo-3 thia-3 eicosatriynamide-5,8,11

A une solution agitée à 0°C, de 1 g de N-(éthyl)thia-3 eicosatriynamide-5,8,11 dans 15 $cm^3$ de chlorure de méthylène, on ajoute goutte à goutte une solution de 0,53 g d'acide métachloroperbenzoïque à 90%, dans 10 $cm^3$ de chlorure de méthylène.

Une heure après, le milieu réactionnel est lavé au bicarbonate de sodium, puis à l'eau. La phase organique est séchée sur sulfate de sodium et déposée sur une colonne de gel de silice.

Le sulfoxyde attendu est élué au mélange chlorure de méthylène-acétate d'éthyle (80-20). Après évaporation de l'éluant, on isole 0,8 g de produit que l'on recristallise dans l00 $cm^3$ d'éther isopropylique.

On obtient 0,5 g de N-(éthyl)oxo-3 thia-3 eicosatriynamide-5,8,ll. Ce sont des cristaux blancs de point de fusion 77°C.

EXEMPLE DE PREPARATION 5

Préparation du N-(éthyl)dioxo-3,3 thia-3 eicosatriynamide-5,8,ll

A une solution agitée à 0°C, de l g de N-(éthyl)thia-3 eicosatriynamide-5,8,ll, dans 20 $cm^3$ de chlorure de méthylène, on ajoute goutte à goutte une solution de 20 $cm^3$ de chlorure de méthylène contenant 2,l équivalents d'acide métachloroperbenzoïque. Après cette introduction, le mélange réactionnel est agité 2 heures à température ambiante, puis lavé à l'aide d'une solution aqueuse de bicarbonate de sodium et enfin à l'eau.

Après élimination du chlorure de méthylène par évaporation sous vide, la sulfone attendue est recristallisée dans l'éther isopropylique en présence d'une trace d'acétate d'éthyle. On obtient 0,75 g de N-(éthyl)dioxo-3,3 thia-3 eicosatriynamide-5,8,ll sous forme de cristaux beiges dont le point de fusion est de 83°C. Les spectres infrarouges et $^lH$ RMN sont conformes à la structure attendue.

Le produit est analysé sous forme de demi-hydrate.

Analyse élémentaire : $C_{21}H_{31}N\ O_3\ S$, 1/2 $H_2O$

| | C | H | N | O | S |
|---|---|---|---|---|---|
| Calculé | 65,25 | 8,34 | 3,62 | 14,48 | 8,29 |
| Trouvé | 65,00 | 7,95 | 3,60 | 14,45 | 8,23 |

EXEMPLE DE PREPARATION 6

Préparation de l'acide dioxo-3,3 thia-3 eicosatriynoïque-5,8,11

A une solution agitée, à 0°C de 6 g d'acide thia-3 eicosatriynoïque-5,8,11 dans 50 $cm^3$ de chlorure de méthylène et 5 $cm^3$ d'acide formique, on ajoute 6 $cm^3$ d'eau oxygénée 110 volumes.

Le mélange est ensuite abandonné pendant 48 heures à la température ordinaire puis concentré à environ 25 $cm^3$ et refroidi à 0°C.

Le produit attendu cristallise. Il est essoré, lavé au dichlorométhane, séché puis recristallisé dans l'acide formique. On obtient 4,5 g d'acide dioxo-3,3 thia-3 eicosatriynoïque-5,8,11 sous forme de cristaux blancs dont le point de fusion est de 132°C.

Analyse élémentaire : $C_{19}H_{26}O_4$ S

| | C | H | O | S |
|---|---|---|---|---|
| Calculé | 65,11 | 7,47 | 18,25 | 9,14 |
| Trouvé | 64,82 | 7,10 | 17,93 | 8,86 |

EXEMPLE DE PREPARATION 7

Préparation du dioxo-3,3 thia-3 eicosatriynoate-5,8,11 de méthyle

Une solution de 1 g d'acide dioxo-3,3 thia-3 eicosatriynoïque-5,8,11 dans 50 $cm^3$ de méthanol est portée sous reflux en présence d'une catalyse acide sulfurique pendant 48 heures. Le mélange est ensuite filtré à température ordinaire. Le méthanol est évaporé sous pression réduite.

Le produit attendu recristallise dans l'éther isopropylique.

On obtient 0,75 g de dioxo-3,3 thia-3 eicosatriynoate-5,8,11 de méthyle, sous forme de paillettes nacrées dont le point de fusion est de 69°C.

EXEMPLE DE PREPARATION 8

Préparation du N-[(hydroxy-2 éthyl)oxyéthyl)] thia-3 eicosatriynamide-5,8,11

A une solution agitée à la température ordinaire sous atmosphère inerte, de 3,18 g d'acide thia-3 eicosatriynoïque-5,8,11 dans 50 $cm^3$ de dichloro-1,2 éthane, on ajoute 3 g de carbonyl diimidazole. Le mélange ainsi obtenu est porté 3 heures à une température comprise entre 40 et 50°C. Puis la solution est refroidie vers 10°C et on ajoute 2,10 g de diglycolamine. 2 heures après la fin de l'introduction, le mélange réactionnel est versé sur une solution saturée de chlorure d'ammonium. La phase organique est décantée, lavée à l'eau, puis séchée sur sulfate de magnésium et enfin traitée au noir animal. Le solvant est éliminé par évaporation sous vide. Le produit obtenu cristallise par refroidissement. Il est recristallisé dans l'éther isopropylique. On isole ainsi 3 g de N-[(hydroxy-2 éthyl)oxyéthyl)] thia-3 eicosatriynamide-5,8,11 sous forme de cristaux blancs-crème de point de fusion 56°C.

Analyse élémentaire : $C_{23}H_{35}NO_3S$

| | C | H | N | O | S |
|---|---|---|---|---|---|
| Calculé | 68,11 | 8,70 | 3,45 | 11,83 | 7,91 |
| Trouvé | 68,06 | 8,68 | 3,46 | 12,01 | 7,88 |

EXEMPLE DE PREPARATION 9

Préparation de l'acide thia-3 eicosatétraynoïque-5,8,II,I4

On prépare séparément le bromo-I heptadécatétrayne-2,5,8,II et le dianion de l'acide thioglycolique.

- Bromo-l heptadécatétrayne-2,5,8,ll.

Un mélange, agité à l'abri de la lumière, de 5 g d'heptadécatétrayne-2,5,8,ll ol-l, de l,25 cm³ de tribromure de phosphore et une goutte de pyridine dans l5 cm³ d'éther anhydre est porté sous reflux pendant 2 heures. La solution est ensuite versée dans l00 cm³ d'eau glacée et extraite deux fois à l'éther éthylique. Les phases éthérées sont lavées avec une solution de bicarbonate de sodium puis à l'eau, et enfin séchées sur sulfate de sodium.

- Dianion de l'acide thioglycolique.

A une solution agitée sous atmosphère inerte de l,6 cm³ d'acide thioglycolique dans 50 cm³ de méthanol, refroidie à 0°C, on ajoute 2,6 g de méthylate de sodium. L'agitation est ensuite maintenue 2 heures à température ambiante.

A cette solution à nouveau refroidie à 0°C, on ajoute la solution éthérée contenant le bromo-1 heptadécatétrayne-2,5,8,11. Une demi-heure après, la réaction est terminée. Le mélange réactionnel est versé sur 200 cm³ d'une solution d'acide sulfurique 1N, la phase éthérée est décantée, lavée à l'eau, séchée sur sulfate de sodium et le solvant évaporé sous pression réduite. Le produit obtenu est agité dans l'heptane. Les cristaux sont essorés et séchés. Les 4,2 g d'acide thia-3 eicosatétraynoïque-5,8,11,14 sont recristallisés dans l'éther isopropylique.

On obtient 3 g de cristaux blanc-crème dont le point de fusion est de 70°C.

Analyse élémentaire : $C_{19}H_{22}O_2S$

| | C | H | O | S |
|---|---|---|---|---|
| Calculé | 72,57 | 7,05 | 10,18 | 10,20 |
| Trouvé | 72,50 | 6,97 | 10,26 | 10,12 |

EXEMPLE DE PREPARATION 10

Préparation du N-[(hydroxy-2 éthyl)oxyéthyl] thia-3 eicosatétraynamide-5,8,11,14

A l'abri de la lumière et sous atmosphère inerte, on ajoute 0,53 g de carbonyl diimidazole à une solution de 0,5 g d'acide thia-3 eicosatétraynoïque-5,8,11,14 dans 20 cm³ de diméthylformamide anhydre. Le mélange est porté à une température de 50°C pendant 2 heures.

Ensuite, à la température ordinaire, on ajoute 0,33 g de diglycolamine et le mélange réactionnel est abandonné une nuit. Le lendemain, il est versé dans 100 cm³ d'eau glacée acidulée (HCl, 1N), extrait trois fois par 50 cm³ de chlorure de méthylène. Les phases organiques sont rassemblées, séchées sur sulfate de sodium et le solvant éliminé sous pression réduite. L'amide attendu est recristallisé dans 60 cm³ d'éther isopropylique.

On obtient 0,5 g de N- [(hydroxy-2 éthyl)oxyéthyl] thia-3 eicosatétraynamide-5,8,11,14 sous forme de cristaux blancs de point de fusion : 57-58°C. Cet amide est analysé sous forme hydraté.

Analyse élémentaire : $C_{23}H_{31}NO_3S$, 1/4 $H_2O$

| | C | H | N | O | S |
|---|---|---|---|---|---|
| Calculé | 68,04 | 7,80 | 3,45 | 12,80 | 7,90 |
| Trouvé | 68,02 | 7,85 | 3,47 | 12,60 | 7,84 |

EXEMPLE DE PREPARATION 11

Préparation de l'acide oxo-3 thia-3 eicosatétraynoïque-5,8,11,14

A une solution de 0,5 g d'acide thia-3 eicosatétraynoïque-5,8,11,14 dans 10 cm³ de dioxane agitée à 10°C, à l'abri de la lumière et sous atmosphère inerte, on ajoute, sous agitation, 10 cm³ d'eau contenant 1 équivalent de bromite de sodium. Au bout de 2 heures, le sulfoxyde précipite. Il est essoré et séché sous pression réduite, puis recristallisé dans l'éther isopropylique.

On obtient 300 mg d'acide oxo-3 thia-3 eicosatétraynoïque-5,8,11,14 sous forme de cristaux blanc-beige

de point de fusion 92°C, analysé sous forme hydraté.
Analyse élémentaire : $C_{19}H_{22}O_3S$, 1/4 $H_2O$

| | C | H | O | S |
|---|---|---|---|---|
| Calculé | 68,12 | 6,78 | 15,52 | 9,57 |
| Trouvé | 68,18 | 6,69 | 15,55 | 9,65 |

EXEMPLE DE PREPARATION 12

Préparation de l'acide dioxo-3,3 thia-3 eicosatétraynoïque-5,8,11,14

A une solution agitée à l'abri de la lumière et sous atmosphère inerte, de 1 g d'acide thia-3 eicosatétraynoïque-5,8,11,14 dans un mélange de 10 cm³ de chlorure de méthylène et 1 cm³ d'acide formique, on ajoute goutte à goutte 1,05 cm³ d'eau oxygénée 9,8 N. L'agitation est maintenue pendant 2 heures puis le mélange est abandonné une nuit à température ordinaire. Le lendemain, le précipité formé est essoré, séché sous pression réduite.

On obtient 0,7 g d'acide dioxo-3,3 thia-3 eicosatétraynoïque-5,8,11,14 sous forme de cristaux blancs, de point de fusion 142°C. Ce produit est analysé sous forme hydratée.
Analyse élémentaire : $C_{19}H_{22}O_4S$, 1/2 $H_2O$

| | C | H | S |
|---|---|---|---|
| Calculé | 65,20 | 6,52 | 9,02 |
| Trouvé | 64,44 | 6,42 | 8,96 |

Les exemples suivants sont destinés à illustrer des compositions pharmaceutiques et/ou cosmétiques conformes à l'invention.

EXEMPLE 1

On prépare la composition suivante :

| | | | |
|---|---|---|---|
| - N-[(hydroxy-2 éthyl)oxyéthyl] thia-3 eicosatriynamide-5,8,11 | | 0,50 | g |
| - Propanol-1 | | 50,00 | g |
| - Propylèneglycol | | 10,00 | g |
| - Hydroxypropylcellulose | | 2,00 | g |
| - Eau | qsp | 100,00 | g |

Cette composition se présente sous forme d'un gel pouvant être appliqué par voie topique.

EXEMPLE 2

On prépare la composition suivante :

| | | | |
|---|---|---|---|
| - (N-hydroxy-2 éthyl) thia-3 eicosa-triynamide-5,8,11 | | 5,00 | g |
| - Polyéthylène micronisé | | 10,00 | g |
| - Myristate d'isopopyle | qsp | 100,00 | g |

Cette composition se présente sous forme d'un onguent hydrophobe destiné à une application topique. On obtient également de bons résultats en remplaçant le (N-hydroxy-2 éthyl)thia-3 eicosatriynamide-5,8,ll par le N-(éthyl)dioxo-3,3 thia-3 eicosatriynamide-5,8,ll.

EXEMPLE 3

On prépare la composition suivante :

| | | | |
|---|---|---|---|
| - N-(éthyl)dioxo-3,3 thia-3 eicosa-triynamide-5,8,11 | | 1,00 | g |
| - Triglycérides des acides caprique, caprylique et stéarique | | 40,00 | g |
| - Triglycérides des acides caprique et caprylique | | 30,00 | g |
| - Vaseline | | 20,00 | g |
| - Huile de vaseline | qsp | 100,00 | g |

Cette composition se présente sous forme d'un onguent hydrophobe destiné à une application topique.

EXEMPLE 4

On prépare la composition suivante :

| | | | |
|---|---|---|---|
| - Acide dioxo-3,3 thia-3 eicosa-triynoïque-5,8,11 | | 0,50 | g |
| - Alcool cétylique | | 6,40 | g |
| - Alcool cétylique oxyéthylené à 20 moles d'oxyde d'éthylène | | 2,10 | g |
| - Monostéarate de glycérol | | 2,00 | g |
| - Triglycérides des acides caprique et caprylique | | 15,00 | g |
| - Propylèneglycol | | 10,00 | g |
| - Eau | qsp | 100,00 | g |

Cette composition se présente sous forme d'une crème destinée à une application topique.

EXEMPLE 5

On prépare la lotion suivante :

| | | | |
|---|---|---|---|
| - Acide dioxo-3,3 thia-3 eicosa-triynoïque-5,8,11 | | 0,10 | g |
| - Ethanol | | 50,00 | g |
| - Propylèneglycol | qsp | 100,00 | g |

Les compositions des exemples 1 à 5 qui précèdent sont toutes fabriquées et stockées en atmosphère inerte et à l'abri de la lumière.

EXEMPLE 6

On prépare la composition suivante :

| | | | |
|---|---|---|---|
| - N-(hydroxy-2 éthyl)thia-3 eicosa-triynamide-5,8,11 | | 0,10 | g |
| - Ethanol absolu | | 1,00 | $cm^3$ |
| - Aromatisant qs, conservateur qs, | | | |
| - Glycérol | qsp | 5,00 | $cm^3$ |

qui est introduite dans une ampoule en verre brun de 5 $cm^3$ destinée à être utilisée par voie orale sous forme d'un soluté buvable.

EXEMPLE 7

On prépare une gélule de 350 mg contenant une poudre ayant la composition suivante :

| | | |
|---|---|---|
| - N-(hydroxy-2 éthyl)oxyéthyl thia-3 eicosatétraynamide-5,8,11,14 | 0,025 | g |
| - Cellulose microcristalline | 0,020 | g |
| - Amidon de maïs | 0,100 | g |
| - Silice colloïdale | 0,020 | g |
| - Stéarate de magnésium | 0,185 | g |

EXEMPLE 8

On prépare un gel sous forme de dispersion vésiculaire ayant la formulation suivante :

| | | |
|---|---|---|
| - Acide thia-3 eicosatriynoïque-5,8,11 | | 0,10 g |
| - Phosphatidylcholine | | 5,00 g |
| - Butylhydroxytoluène | | 0,10 g |
| - Acide polyacrylique réticulé vendu sous le nom de CARBOPOL 940 par la Société GOODRICH | | 0,50 g |
| - Parahydroxybenzoate de méthyle | | 0,01 g |
| - NaOH dilué qs | | pH = 7,4 |
| - NaCl à 0,9% | qsp | 100,00 g |

Le procédé de préparation comporte les étapes suivantes :

1°) Le parahydroxybenzoate de méthyle est solubilisé dans une solution de NaCl à 0,9%.

2°) Le butylhydroxytoluène est dissous dans quelques ml de $CHCl_3$ ; on y ajoute la phosphatidylcholine, puis on complète la quantité de $CHCl_3$ à 60 ml. L'ensemble est mis sous agitation magnétique jusqu'à solubilisation: on ajoute l'acide thia-3-eicosatriynoïque-5,8,11 et on agite de nouveau jusqu'à solubilisation. On évapore ensuite le $CHCl_3$ en portant à sec la phase lipidique dans un évaporateur rotatif.

3°) La solution obtenue au cours de la première étape, est ajoutée, sous agitation mécanique appropriée (3 heures à 200tr/mn et à 20°C), à la phase lipidique, obtenue dans la deuxième étape. Il en résulte une dispersion laiteuse et homogène de vésicules ayant un diamètre de 0,5 um à 5 um.

4°) Le Carbopol 940 est ajouté en pluie, sous agitation, à la dispersion ainsi obtenue et le pH du gel est ajusté à 7,4 avec de la soude diluée.

## Revendications

**1.** Composé caractérisé par le fait qu'il répond à la formule générale (I) :

$$C_5H_{11}\text{-}A\text{-}CH_2\text{-}C\equiv C\text{-}CH_2\text{-}C\equiv C\text{-}CH_2\text{-}C\equiv C\text{-}CH_2\text{-}\underset{\downarrow \atop (O)_n}{S}\text{-}CH_2\text{-}COR$$

dans laquelle :

A correspond aux radicaux suivants :

$$-(CH_2)_2- \text{ ou } -(C\equiv C)-;$$

n est égal à 0,1 ou 2;

R représente un radical hydroxyle, alcoxy de formule $-OR_1$, ou un radical amino de formule :

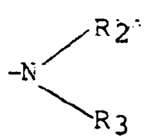

$R_1$ représente un radical alkyle ayant de 1 à 20 atomes de carbone, monohydroxyalkyle, polyhydroxyalkyle, aryle ou aralkyle, éventuellement substitué(s) ou un reste de sucre;

$R_2$ et $R_3$ représentent, indépendamment l'un de l'autre, un atome d'hydrogène, un radical alkyle

inférieur, un radical monohydroxyalkyle éventuellement interrompu par un hétéroatome, un radical polyhydroxyalkyle, un radical aryle ou benzyle, éventuellement substitué(s), un reste d'amino acide ou de sucre aminé;

$R_2$ et $R_3$ pris ensemble forment également avec l'atome d'azote auquel ils sont rattachés, un hétérocycle comportant éventuellement un hétéroatome supplémentaire et/ou étant éventuellement substitué par un groupement alkyle ou hydroxyalkyle,

et les sels desdits composés ainsi que leurs isomères optiques et géométriques.

2. Composés selon la revendication 1, caractérisés par le fait que dans la formule générale (I) le groupement alkyle désigne un radical choisi parmi les radicaux méthyle, éthyle, isopropyle, butyle, tertiobutyle, éthyl-2 hexyle, isooctyle, dodécylé, hexadécyle et octadécyle; que le radical monohydroxyalkyle éventuellement interrompu par un hétéroatome désigne un radical ayant 2 à 6 atomes de carbone, éventuellement interrompu par un atome d'oxygène; que le radical polyhydroxyalkyle désigne un radical ayant 3 à 6 atomes de carbone et 2 à 5 groupes hydroxyle; que le radical aryle est un radical phényle, éventuellement substitué par au moins un atome d'halogène, un groupement -OH, $-NO_2$, un radical alkyle inférieur, un radical trifluorométhyle ou une fonction acide carboxylique; que le radical aralkyle est choisi parmi le radical benzyle ou phénétyle; qu'un reste de sucre est un reste choisi parmi les restes dérivant du glucose, du mannose, de l'érythrose ou du galactose; que les restes de sucres aminés sont choisis parmi les restes de glucosamine, de galactosamine, de mannosamine ou de méglumine; que les hétérocycles formés par $R_2$ et $R_3$ avec l'atome d'azote auquel ils sont rattachés, sont choisis parmi les radicaux pipéridino, pipérazino, morpholino, pyrrolidino ou (hydroxy-2 éthyl)-4 pipérazino.

3. Composés selon la revendication 1 ou 2, caractérisés par le fait que les sels sont choisis parmi les sels de métaux alcalins, alcalino-terreux, de zinc, d'une amine organique, lorsqu'ils comportent au moins une fonction acide libre, ou parmi les sels d'un acide minéral ou organique choisis parmi les chlorhydrates, les bromhydrates ou les citrates, lorsqu'ils comportent au moins une fonction amine.

4. Composés selon l'une quelconque des revendications 1 à 3, caractérisés par le fait qu'il s'agit de l'acide thia-3 eicosatriynoïque-5,8,11, l'acide thia-3 eicosatétraynoïque-5,8,11,14, l'acide oxo-3 thia-3 eicosatriynoïque-5,8,11, l'acide oxo-3 thia-3 eicosatétraynoïque-5,8,11,14, l'acide dioxo-3,3 thia-3 eicosatriynoïque-5,8,11, l'acide dioxo-3,3 thia-3 eicosatétraynoïque-5,8,11,14, ainsi que les amides de ceux-ci.

5. Procédé de préparation de l'acide thia-3 eicosatriynoïque-5,8,11, caractérisé par le fait que dans une première étape on fait réagir l'halogéno-1 tétradécadiyne-2,5 répondant à la formule (2) :

$$C_8H_{17}\text{-}C{\equiv}C\text{-}CH_2\text{-}C{\equiv}C\text{-}CH_2X \qquad (\underline{2})$$

avec un dianion de l'alcool propargylique pour préparer l'heptadécatridiyne-2,5,8 ol-1, répondant à la formule (3) :

$$C_8H_{17}\text{-}C{\equiv}C\text{-}CH_2\text{-}C{\equiv}C\text{-}CH_2\text{-}C{\equiv}C\text{-}CH_2OH \qquad (\underline{3})$$

que dans une deuxième étape, on traite le composé (3) par un trihalogénure de phosphore dans un solvant chloré pour préparer l'halogéno-1 heptadécatriyne-2,5,8 de formule (4) :

$$C_8H_{17}\text{-}C{\equiv}C\text{-}CH_2\text{-}C{\equiv}C\text{-}CH_2\text{-}C{\equiv}C\text{-}CH_2X \qquad (\underline{4})$$

et que dans une troisième étape, on fait réagir le composé de formule (4) avec le dianion de l'acide thioglycolique pour préparer l'acide thia-3 eicosatriynoïque-5,8,11.

6. Procédé de préparation de l'acide thia-3 eicosatétraynoïque-5,8,11,14, caractérisé par le fait que l'on fait réagir dans une première étape l'halogéno-1 undécadiyne-2,5 de formule (7) :

$$C_5H_{11}\text{-}C{\equiv}C\text{-}CH_2\text{-}C{\equiv}C\text{-}CH_2X \qquad (\underline{7})$$

avec le dianion de l'alcool propargylique pour préparer l'hydroxy-1 tétradécatriyne-2,5,8 de formule (8) :

$$C_5H_{11}\text{-}C{\equiv}C\text{-}CH_2\text{-}C{\equiv}C\text{-}CH_2\text{-}C{\equiv}C\text{-}CH_2OH \qquad (\underline{8})$$

que l'on transforme, dans une deuxième étape, l'alcool de formule (8) en halogéno-1 tétradécatriyne-2,5,8 de formule (9) :

$$C_5H_{11}(C{\equiv}C\text{-}CH_2)_3\text{-}X \qquad (\underline{9})$$

par action d'un trihalogénure de phosphore en milieu solvant chloré;

que l'on fait agir dans une troisième étape un dianion de l'alcool propargylique sur l'halogénure de formule (9) pour préparer l'heptadécatétrayne-2,5,8,11 ol-1 de formule (10) :

$$C_5H_{11}(C{\equiv}C\text{-}CH_2)_4\text{-}OH \qquad (\underline{10})$$

que dans une quatrième étape on transforme ce dernier en halogéno-1 heptadécatétrayne-2,5,8,11 de formule (11) :

$C_5H_{11}(C\equiv C-CH_2)_4-X$ (11)

par action du trihalogénure de phosphore et que dans une cinquième étape on amène à faire réagir l'halogéno-1 heptadécatétrayne-2,5,8,11 de formule (11) avec le dianion de l'acide thioglycolique pour former l'acide thia-3 eicosatétraynoïque-5,8,11,14.

**7.** Procédé de préparation d'esters de formule (II) :

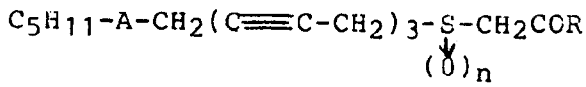

(II)

dans laquelle $R=OR_1$ et $A,R_1$ et n sont tels que définis dans la revendication 1, caractérisé par le fait que l'on fait agir sur le composé de formule (I') :

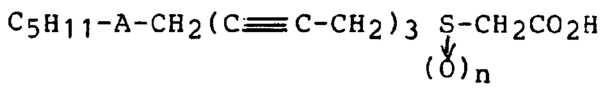

(I')

un alcool de formule $HOR_1$ en milieu acide ou qu'on procède à une réaction de déplacement de l'halogène d'un halogénure d'alkyle $XR_1$par réaction du carboxylate de sodium ou de potassium de (I').

**8.** Procédé de préparation des amides de formule (III) :

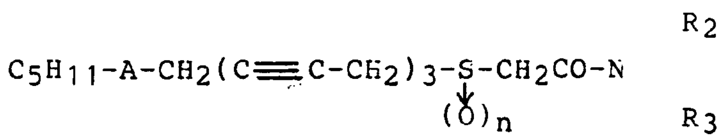

(III)

dans laquelle $A,n,R_2,R_3$ ont les significations indiquées dans la revendication 1, caractérisé par le fait que l'on fait réagir, en milieu solvant, une forme activée du composé de formule (I') :

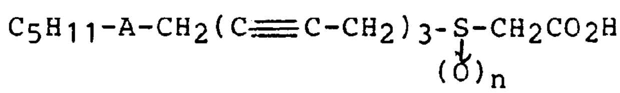

(I')

sur une amine de formule :

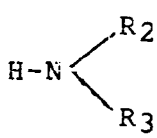

dans laquelle $R_2$ et $R_3$ ont la signification indiquée ci-dessus.

**9.** Procédé de préparation des amides répondant à la formule (III) :

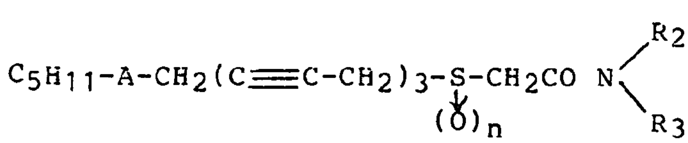

(III)

dans laquelle $A,n,R_2$ et $R_3$ ont les significations indiquées dans revendication 1, caractérisé par le fait que

l'on traite l'halogéno-1 heptadécatriyne-2,5,8 de formule (4) :

$$C_8H_{17}\text{-}C\equiv C\text{-}CH_2\text{-}C\equiv C\text{-}CH_2\text{-}C\equiv C\text{-}CH_2X \qquad (\underline{4})$$

ou bien l'halogéno-1 heptadécatétrayne-2,5,8,11 de formule (11) :

$$C_5H_{11}(C\equiv C\text{-}CH_2)_4\text{-}X \qquad (\underline{11})$$

par le thiolate préparé par action d'une amine de formule :

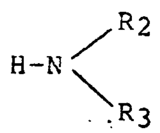

dans laquelle $R_2$ et $R_3$ ont les significations indiquées dans la revendication 1 sur le thioglycolate d'éthyle.

**10.** Procédé de préparation des sulfoxydes et des sulfones répondant à la formule générale (I), dans laquelle n est égal à 1 ou 2, caractérisé par le fait que l'on fait réagir sur les thioéthers préparés selon l'un quelconque des procédés définis dans l'une quelconque des revendications 5 à 9, avec 1 ou 2 équivalents d'un peracidé organique dans un solvant organique.

**11.** Médicament caractérisé par le fait qu'il contient un composé tel que défini dans l'une quelconque des revendications 1 à 4.

**12.** Composition pharmaceutique destinée à être administrée par voie systémique ou par voie locale, caractérisée par le fait qu'elle contient en présence d'un support ou d'un diluant pharmaceutiquement acceptable au moins un composé tel que défini dans l'une quelconque des revendications 1 à 4.

**13.** Composition pharmaceutique destinée à être administrée par voie topique, se présentant sous forme de crème, de teinture, d'onguent, de pommade, de poudre, de timbre, de tampon imbibé, de solution, de gel, de lotion, de spray, de shampooing, de suspension, de microdispersion de vésicules lipidiques, ioniques ou non ioniques, caractérisée par le fait qu'elle contient au moins un composé tel que défini dans l'une quelconque des revendications 1 à 4.

**14.** Composition pharmaceutique destinée à être administrée par voie occulaire se présentant sous forme de collyre ou de pommade, caractérisée par le fait qu'elle contient au moins un composé tel que défini dans l'une des revendications 1 à 4.

**15.** Composition pharmaceutique destinée à être administrée par voie aérienne se présentant sous forme de pulvérisations, de solutions ou de suspensions, caractérisée par le fait qu'elle contient au moins un composé tel que défini dans l'une des revendications 1 à 4.

**16.** Composition pharmaceutique se présentant sous forme de composition destinée à être administrée de façon parentérale par voie intraveineuse, intrapéritonéale, intramusculaire, sous-cutanée ou intradermique, caractérisée par le fait qu'elle contient un composé tel que défini dans l'une quelconque des revendications 1 à 4, dans un diluant pharmaceutiquement acceptable.

**17.** Composition pharmaceutique destinée à être administrée par voie entérale, se présentant sous forme de comprimé, de gélule, de dragée, de suppositoire, de sirop, de suspension, de dispersion, de solution, de poudre, de granulé, d'émulsion, caractérisée par le fait qu'elle contient au moins un composé tel que défini dans l'une quelconque des revendications 1 à 4.

**18.** Composition pharmaceutique selon l'une quelconque des revendications 12 à 17, caractérisée par le fait que le composé actif de formule (I) est présent dans des proportions comprises entre 0,01 et 10% en poids par rapport au poids total de la composition, et de préférence entre 0,1 et 5% en poids.

**19.** Composition pharmaceutique selon la revendication 12 ou 13, caractérisée par le fait que le composé de formule (I) est associé à un agent hydratant, antiséborrhéique, antibiotique, anti-inflammatoire, antipsoriasique, un agent favorisant la repousse des cheveux, un caroténoïde, un inhibiteur de phospholipase $A_2$ ou un antifongique.

**20.** Utilisation d'un composé tel que défini dans l'une quelconque des revendications 1 à 4 pour la préparation d'un médicament destiné au traitement et à la prophylaxie des maladies allergiques et dans le traitement des dermatoses et des maladies inflammatoires tels que le psoriasis, l'eczéma, l'acné.

**21.** Utilisation selon la revendication 20 d'un composé tel que défini dans l'une quelconque des revendications 1 à 4, pour la préparation d'un médicament destiné au traitement et à la prophylaxie des maladies allergiques, au traitement des dermatoses et des maladies inflammatoires tels que le psoriasis, l'eczéma, l'acné, à des doses de 0,05 à 500 mg/kg/jour et de préférence 0,5 à 100 mg/kg/jour.

**22.** Composition cosmétique caractérisée par le fait qu'elle contient au moins un composé tel que défini dans l'une quelconque des revendications 1 à 4, dans des proportions comprises entre 0,01 et 10% en poids, et de préférence comprises entre 0,1 et 5% en poids par rapport au poids total de la composition, en présence d'un support cosmétiquement acceptable.

**23.** Application d'un composé tel que défini dans l'une quelconque des revendications 1 à 4, comme produit cosmétique.

**Patentansprüche**

I. Verbindung der allgemeinen Formel (I):

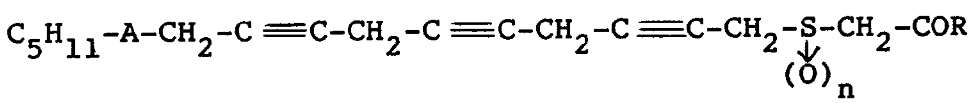

worin:

A die folgenden Reste darstellt:

$-(CH_2)_2-$ oder $-(C{\equiv}C)-$;

n = 0,I oder 2 ist;

R eine Hydroxylgruppe, einen Alkoxyrest der Formel $-OR_I$ oder einen Aminorest der Formel darstellt:

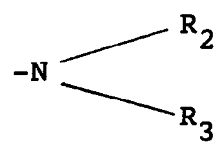

$R_I$ einen Alkylrest mit I bis 20 Kohlenstoffatomen, Monohydroxyalkyl-, Polyhydroxyalkyl-, Aryl- oder Aralkylrest, gegebenenfalls substituiert, oder einen Zuckerrest darstellt;

$R_2$ und $R_3$, unabhängig voneinander, ein Wasserstoffatom, eine Niedrigalkyl-, einen gegebenenfalls durch ein Heteroatom unterbrochenen Monohydroxyalkyl-, einen Polyhydroxyalkyl-, Aryl- oder Benzylrest, gegebenenfalls substituiert, einen Rest einer Aminosäure oder eines aminierten Zuckers darstellen;

$R_2$ und $R_3$ zusammen mit dem Stickstoffatom, an das sie gebunden sind, auch einen Heterozyklus bilden, der gegebenenfalls ein weiteres Heteroatom aufweist und/oder gegebenenfalls durch eine Alkyl- oder Hydroxyalkylgruppe substituiert ist,

sowie die Salze der genannten Verbindungen und deren optische und geometrische Isomere.

2. Verbindungen gemäß Anspruch I, dadurch **gekennzeichnet**, daß in der allgemeinen Formel (I) die Alkylgruppe einen Rest bedeutet, ausgewählt aus Methyl-, Ethyl-, Isopropyl-, Butyl-, t-Butyl-, 2-Ethylhexyl-, Isooctyl-, Dodecyl-, Hexadecyl- und Octadecylresten; daß der gegebenenfalls durch ein Heteroatom unterbrochene Monohydroxyalkylrest einen, gegebenenfalls durch ein Sauerstoffatom unterbrochenen, Rest mit 2 bis 6 Kohlenstoffatomen darstellt; daß der Polyhydroxyalkylrest einen Rest mit 3 bis 6 Kohlenstoffatomen und 2 bis 5 Hydroxylgruppen darstellt; daß der Arylrest ein Phenylrest ist, gegebenenfalls substituiert durch mindestens ein Halogenatom, eine -OH-, $-NO_2$-Gruppe, einen Niedrigalkylrest, einen Trifluormethylrest oder eine Carboxylsäurefunktion; daß der Aralkylrest aus dem Benzyl- oder Phenethylrest ausgewählt ist; daß der Zuckerrest ein Rest ist, ausgewählt aus Resten, die sich von Glukose, Mannose, Erythrose oder Galactose ableiten; daß die Reste der aminierten Zucker aus Resten von Glukosamin, Galactosamin, Mannosamin oder Meglumin ausgewählt sind; und daß die durch $R_2$ und $R_3$ mit dem Stickstoffatom, an das sie gebunden sind, gebildeten Heterozyklen aus Piperidino-, Piperazino-, Morpholino-, Pyrrolidino- oder 4-(2-Hydroxyethyl)piperazinoresten ausgewählt sind.

3. Verbindungen gemäß Anspruch I oder 2, dadurch **gekennzeichnet**, daß die Salze aus Alkalimetall-, Erdalkalimetall-, Zink-, organischen Aminsalzen, wenn die Verbindungen mindestens eine freie Säurefunktion aufweisen, oder aus Salzen einer Mineralsäure oder einer organischen Säure ausgewählt sind, ausgewählt aus Hydrochloriden, Hydrobromiden oder Zitraten, wenn die Verbindungen mindestens eine Aminfunktion aufweisen.

4. Verbindungen gemäß jedem der Ansprüche I bis 3, dadurch **gekennzeichnet** , daß sie
3-Thia-eicosatriin-5,8,II-säure,
3-Thia-eicosatetrain-5,8,II,I4-säure,
3-Oxo-3-thia-eicosatriin-5,8,II-säure,
3-Oxo-3-thia-eicosatetrain-5,8,II,I4-säure,
3,3-Dioxo-3-thia-eicosatriin-5,8,II-säure,
3,3-Dioxo-3-thia-eicosatetrain,5,8,II,I4-säure sowie die Amide derselben sind.

5. Verfahren zur Herstellung von 3-Thia-eicosatriin-5,8,II-säure, dadurch **gekennzeichnet**, daß man in einer ersten Sufe I-Halogentetradecadiin-2,5 der Formel (2):

$$C_8H_{17}\text{-}C{\equiv}C\text{-}CH_2\text{-}C{\equiv}C\text{-}CH_2X \qquad (2)$$

mit einem Dianion des Propargylalkohols reagieren läßt, um Heptadecatriin-2,5,8-ol-I der Formel (3) herzustellen:

$$C_8H_{I7}\text{-}C \equiv C\text{-}CH_2\text{-}C \equiv C\text{-}CH_2\text{-}C \equiv C\text{-}CH_2OH \qquad (3)$$

daß man in einer zweiten Stufe die Verbindung (3) mit einem Phosphortrihalogenid in einem chlorierten Lösungsmittel behandelt, um
I-Halogenheptadecatriin-2,5,8 der Formel (4) herzustellen:

$$C_8H_{I7}\text{-}C{\equiv}\ C\text{-}CH_2\text{-}C \equiv C\text{-}CH_2\text{-}C \equiv C\text{-}CH_2X \qquad (4)$$

und daß man in einer dritten Stufe die Verbindung der Formel (4) mit dem Dianion der Thioglycolsäure reagieren läßt, um die
3-Thia-eicosatriin-5,8,II-säure herzustellen.

6. Verfahren zur Herstellung der 3-Thia-eicosatetrain-5,8,II,I4-säure, dadurch **gekennzeichnet**, daß man in einer ersten Stufe I-Halogenundecadiin-2,5 der Formel (7):

$$C_5H_{II}\text{-}C \equiv C\text{-}CH_2\text{-}C{\equiv}C\text{-}CH_2X \qquad (7)$$

mit dem Dianion des Propargylalkohols reagieren läßt, um I-Hydroxytetradecatriin-2,5,8 der Formel (8) herzustellen:

$$C_5H_{II}\text{-}C{\equiv}C\text{-}CH_2\text{-}C \equiv C\text{-}CH_2\text{-}C{\equiv}C\text{-}CH_2OH \qquad (8)$$

daß man in einer zweiten Stufe den Alkohol der Formel (8) in I-Halogentetradecatriin-2,5,8 der Formel (9) durch ein Phosphortrihalogenid in einem chlorierten Lösungsmittel überführt:

$$C_5H_{II}(C \equiv C\text{-}CH_2)_3\text{-}X \qquad (9)$$

daß man in einer dritten Stufe ein Dianion des Propargylalkohols mit dem Halogenid der Formel (9) reagieren läßt, um Heptadecatetrain-2,5,8,II-ol-I der Formel (10) herzustellen:

$$C_5H_{II}(C \equiv C\text{-}CH_2)_4\text{-}OH \qquad (I0)$$

daß man in einer vierten Stufe diese Verbindung in I-Halogenheptadecatetrain-2,5,8,II der Formel (II) durch Reaktion mit Phosphortrihalogenid überführt:

$$C_5H_{II}(C \equiv C\text{-}CH_2)_4\text{-}X \qquad (II)$$

und daß man in einer fünften Stufe das I-Halogenheptadecatetrain-2,5,8,II der Formel (II) mit einem Dianion der Thioglycolsäure reagieren läßt, um 3-Thia-eicosatetrain-5,8,II,I4-säure herzustellen.

7. Verfahren zur Herstellung von Estern der Formel (II):

$$C_5H_{11}\text{-}A\text{-}CH_2(C{\equiv}C\text{-}CH_2)_3\text{-}\underset{\underset{(O)_n}{\downarrow}}{S}\text{-}CH_2\text{-}COR \qquad (II)$$

worin R = $OR_I$ und A, $R_I$ und n wie in Anspruch I definiert sind, dadurch
**gekennzeichnet**, daß man einen Alkohol der Formel $HOR_I$ in saurem Medium mit der Verbindung der Formel (I′) reagieren läßt:

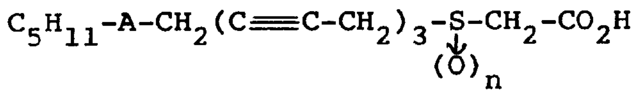
(I')

oder daß man eine Halogenaustauschreaktion am Alkylhalogenid $XR_I$ durch Reaktion mit dem Natrium- oder Kaliumcarboxylat von (I') durchführt.

8. Verfahren zur Herstellung der Amide von Formel (III):

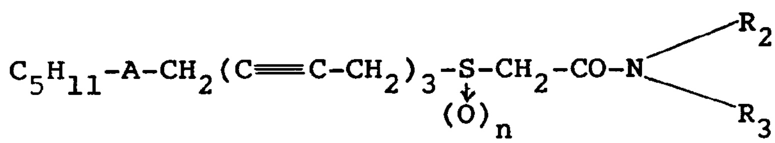
(III)

worin A, n, $R_2$, $R_3$ die in Anspruch I angegebenen Bedeutungen haben, dadurch **gekennzeichnet**, daß man in einem Lösungsmittel eine aktivierte Form der Verbindung der Formel (I'):

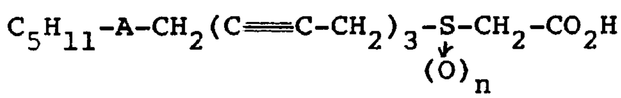
(I')

mit einem Amin der Formel reagieren läßt:

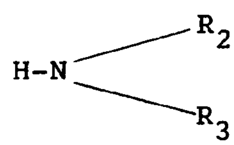

worin $R_2$ und $R_3$ die oben angegebene Bedeutung haben.

9. Verfahren zur Herstellung der Amide der Formel (III):

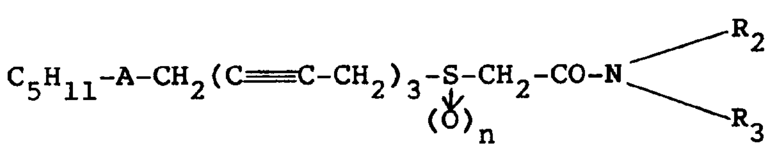
(III)

worin A, n, $R_2$ und $R_3$ die in Anspruch I angegebenen Bedeutungen haben, dadurch **gekennzeichnet**, daß man I-Halogenheptadecatriin-2,5,8 der Formel (4):

$C_8H_{I7}$-C ≡ C-$CH_2$-C ≡C-$CH_2$-C≡ C-$CH_2$X (4)

oder auch I-Halogenheptadecatetrain-2,5,8,11 der Formel (II):

$C_5H_{II}$(C≡ C-$CH_2$)$_4$-X (II)

mit dem Thiolat behandelt, das hergestellt wird, indem man Ethylthioglycolat mit einem Amin der Formel reagieren läßt:

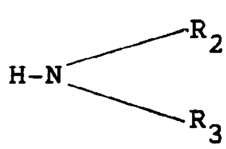

worin $R_2$ und $R_3$ die in Anspruch I angegebenen Bedeutungen haben.

I0. Verfahren zur Herstellung von Sulfoxiden und Sulfonen der allgemeinen Formel (I), worin n = I oder 2 ist, dadurch **gekennzeichnet**, daß man die Thioether, erhältlich gemäß eines jeden der Verfahren gemäß eines

jeden der Ansprüche 5 bis 9 mit I oder 2 Äquivalenten einer organischen Persäure in einem organischen Lösungsmittel reagieren läßt.

II. Medikament, dadurch **gekennzeichnet**, daß es eine Verbindung gemäß eines jeden der Ansprüche I bis 4 enthält.

I2. Pharmazeutische Zusammensetzung zur systemischen oder lokalen Verabreichung, dadurch **gekennzeichnet**, daß sie zusammen mit einem pharmazeutisch zulässigen Träger oder Verdünnungsmittel mindestens eine Verbindung gemäß eines jeden der Ansprüche I bis 4 enthält.

I3. Pharmazeutische Zusammensetzung zur topischen Verabreichung in Form einer Creme, Tinktur, Salbe, Pommade, eines Pulvers, Puders, getränkten Tampons, einer Lösung, eines Gels, einer Lotion, eines Sprays, eines Shampoos, einer Suspension, einer Mikrodispersion aus lipiden, ionischen oder nicht-ionischen Bläschen, dadurch **gekennzeichnet**, daß sie mindestens eine Verbindung gemäß eines jeden der Ansprüche I bis 4 enthält.

I4. Pharmazeutische Zusammensetzung für die Augen in Form. eines Augenwassers oder einer Augensalbe, dadurch **gekennzeichnet**, daß sie mindestens eine Verbindung gemäß eines jeden der Ansprüche I bis 4 enthält.

I5. Pharmazeutische Zusammensetzung zur Verabreichung auf dem Luftweg in Form von Pulvern, Lösungen oder Suspensionen, dadurch **gekennzeichnet**, daß sie mindestens eine Verbindung gemäß eines jeden der Ansprüche I bis 4 enthält.

I6. Pharmazeutische Zusammensetzung in Form einer Zusammensetzung zur Parenteralen Verabreichung, und zwar intravenös, intraperitoneal, intramuskulär, subkutan oder intradermisch, dadurch **gekennzeichnet**, daß sie eine Verbindung gemäß eines jeden der Ansprüche I bis 4 in einem pharmazeutisch zulässigen Verdünnungsmittel enthält.

I7. Pharmazeutische Zusammensetzung zur enteralen Verabreichung, in Form einer Tablette, einer Kapsel, eines Dragees, Suppositoriums, Sirups, einer Suspension, Dispersion, Lösung, eines Pulvers, Körnchens, einer Emulsion, dadurch **gekennzeichnet**, daß sie mindestens eine Verbindung gemäß eines jeden der Ansprüche I bis 4 enthält.

I8. Pharmazeutische Zusammensetzung gemäß eines jeden der Ansprüche I2 bis I7, dadurch **gekennzeichnet**, daß die Wirkverbindung der Formel (I) in Mengenverhältnissen von 0,0I bis I0 Gew.%, vorzugsweise 0,I bis 5 Gew.%, bezogen auf das Gesamtgewicht der Zusammensetzung, vorliegt.

I9. Pharmazeutische Zusammensetzung gemäß Anspruch I2 oder I3, dadurch **gekennzeichnet**, daß die Verbindung der Formel (I) zusammen mit einem Hydratisiermittel, einem antiseborrheischen, antibiotischen, entzündungshemmenden, antipsoriasischen Mittel, einem Mittel, das das Wiederauftteten des Haarwachstums fördert, einem Carotinoid, einem Inhibitor der Phospholipase $A_2$ oder einem Fungizid vorliegt.

20. Verwendung einer Verbindung gemäß eines jeden der Ansprüche I bis 4, zur Herstellung eines Medikaments zur Behandlung und Prophylaxe allergischer Krankheiten und zur Behandlung von Dermatosen und Entzündungen, wie Psoriasis, Ekzemen, Akne.

2I. Verwendung gemäß Anspruch 20 einer Verbindung gemäß eines jeden der Ansprüche I bis 4 zur Herstellung eines Medikaments zur Behandlung und Prophylaxe allergischer Krankheiten, zur Behandlung von Dermatosen und Entzündungen, wie Psoriasis, Ekzemen, Akne, in Dosismengen von 0,05 bis 500 mg/kg/Tag, vorzugsweise 0,5 bis I00 mg/kg/Tag.

22. Kosmetische Zusammensetzung, dadurch **gekennzeichnet**, daß sie mindestens eine Verbindung gemäß einem der Ansprüche I bis 4 in Mengenverhältnissen von 0,0I bis I0 Gew.%, vorzugsweise 0,I bis 5 Gew.%, bezogen auf das Gesamtgewicht der Zusammensetzung, zusammen mit einem kosmetisch verträglichen Träger enthält.

23. Anwendung einer Verbindung gemäß eines jeden der Ansprüche I bis 4 als Kosmetikprodukt.

**Claims**

**1.** Compound characterized in that it corresponds to the general formula (I):

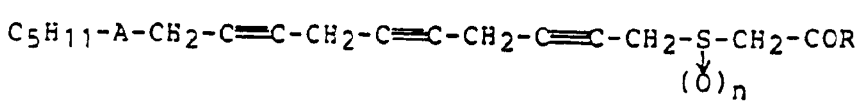

in which:

A corresponds to the following radicals:

$-(CH_2)_2-$ or $-(C\equiv C)-$;
n is equal to 0, 1 or 2;
R denotes a hydroxyl or alkoxy radical of formula $-OR_1$, or an amino radical of formula:

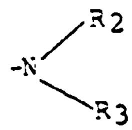

$R_1$ denotes an alkyl containing from 1 to 20 carbon atoms, monohydroxyalkyl, polyhydroxyalkyl, aryl or aralkyl radical, optionally substituted, or a sugar residue;

$R_2$ and $R_3$, independently of each other, denote a hydrogen atom, a lower alkyl radical, a monohydroxyalkyl radical optionally interrupted by a heteroatom, a polyhydroxyalkyl radical, an aryl or benzyl radical, optionally substituted, an amino acid residue or an amino sugar residue;

$R_2$ and $R_3$ taken together, together with the nitrogen atom to which they are linked, also form a heterocyclic ring optionally containing an additional heteroatom and/or optionally substituted by an alkyl or hydroxyalkyl group,

and the salts of the said compounds and their optical and geometric isomers.

2. Compounds according to Claim 1, characterized in that in the general formula (I) the alkyl group denotes a radical chosen from the methyl, ethyl, isopropyl, butyl, tert-butyl, 2-ethylhexyl, isooctyl, dodecyl, hexadecyl and octadecyl radicals; that the monohydroxyalkyl radical optionally interrupted with a heteroatom denotes a radical containing 2 to 6 carbon atoms, optionally interrupted with an oxygen atom; that the polyhydroxyalkyl radical denotes a radical containing 3 to 6 carbon atoms and 2 to 5 hydroxyl groups; that the aryl radical is a phenyl radical, optionally substituted by at least one halogen atom, an -OH, $-NO_2$ group, a lower alkyl radical, a trifluoromethyl radical or a carboxylic acid functional group; that the aralkyl radical is chosen from the benzyl or phenethyl radical; that a sugar residue is a residue chosen from the residues derived from glucose, mannose, erythrose or galactose; that the amino sugar residues are chosen from the residues of glucosamine, galactosamine, mannosamine or meglumine; that the heterocyclic rings formed by $R_2$ and $R_3$ with the nitrogen atom to which they are linked are chosen from piperidino, piperazino, morpholino, pyrrolidino or 4-(2-hydroxyethyl)piperazino radicals.

3. Compounds according to Claim 1 or 2, characterized in that the salts are chosen from the salts of alkali or alkaline-earth metals, of zinc, or of an organic amine, when they contain at least one free acidic functional group, or from the salts of an inorganic or organic acid, chosen from hydrochlorides, hydrobromides or citrates, when they contain at least one amine functional group.

4. Compounds according to any one of Claims 1 to 3, characterized in that they are 3-thia-5,8,11-eicosatriynoic acid, 3-thia-5,8,11,14-eicosatetraynoic acid, 3-oxo-3-thia-5,8,11-eicosatriynoic acid, 3-oxo-3-thia-5,8,11,14-eicosatetraynoic acid,3,3-dioxo-3-thia-5,8,11-eicosatriynoic acid, 3,3-dioxo-3-thia-5,8,11,14-eicosatetraynoic acid and the amides of these.

5. Process for the preparation of 3-thia-5,8,11-eicosatriynoic acid, characterized in that, in a first stage, the 1-halo-2,5-tetradecadiyne corresponding to the formula (2):

$$C_8H_{17}\text{-}C\equiv C\text{-}CH_2\text{-}C\equiv C\text{-}CH_2X \qquad (2)$$

is reacted with a dianion of propargyl alcohol to prepare 2,5,8-heptadecatriyn-1-ol corresponding to the formula (3) :

$$C_8H_{17}\text{-}C\equiv C\text{-}CH_2\text{-}C\equiv C\text{-}CH_2\text{-}C\equiv C\text{-}CH_2OH \qquad (3)$$

that, in a second stage, the compound (3) is treated with a phosphorus trihalide in a chlorinated solvent to prepare the 1-halo-2,5,8-heptadecatriyne of formula (4):

$$C_8H_{17}\text{-}C\equiv C\text{-}CH_2\text{-}C\equiv C\text{-}CH_2\text{-}C\equiv C\text{-}CH_2X \qquad (4)$$

and that, in a third stage, the compound of formula (4) is reacted with the dianion of thioglycolic acid to prepare 3-thia-5,8,11-eicosatriynoic acid.

6. Process for the preparation of 3-thia-5,8,11,14-eicosatetraynoic acid, characterized in that, in a first stage, the 1-halo-2,5-undecadiyne of formula (7):

$$C_5H_{11}\text{-}C\equiv C\text{-}CH_2\text{-}C\equiv C\text{-}CH_2X \qquad (7)$$

is reacted with the dianon of propargyl alcohol to prepare 1-hydroxy-2,5,8-tetradecatriyne of formula (8):

$$C_5H_{11}-C\equiv C-CH_2-C\equiv C-CH_2-C\equiv C-C-CH_2OH \qquad (8)$$

that the alcohol of formula (8) is converted, in a second stage, into 1-halo-2,5,8-tetradecatriyne of formula (9):

$$C_5H_{11}(C\equiv C-CH_2)_3-X \qquad (9)$$

by the action of phosphorus trihalide in a chlorinated solvent medium;
that, in a third stage, a dianion of propargyl alcohol is reacted with the halide of formula (9) to prepare 2,5,8,11-heptadecatetrayn-1-ol of formula (10) :

$$C_5H_{11}(C\equiv C-CH_2)_4-OH \qquad (10)$$

that, in a fourth stage, the latter is converted into 1-halo-2,5,8,11-heptadecatetrayne of formula (11):

$$C_5H_{11}(C\equiv C-CH_2)_4-X \qquad (11)$$

by the action of the phosphorus trihalide and that, in a fifth stage, the 1-halo-2,5,8,11-heptadecatetrayne of formula (11) is made to react with the dianion of thioglycolic acid to form 3-thia-5,8,11,14-eicosatetraynoic acid.

7. Process for the preparation of esters of formula (II):

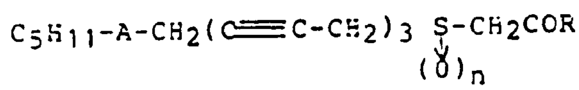

(II)

in which R=OR$_1$ and A, R$_1$ and n are such as defined in Claim 1, characterized in that the compound of formula (I') :

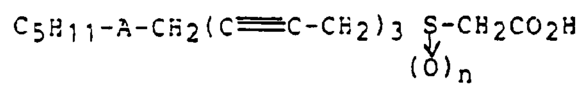

(I')

is reacted with an alcohol of formula HOR$_1$ in an acidic medium or that a halogen displacement reaction of an alkyl halide XR$_1$ is performed by reaction with a sodium or potassium carboxylate.

8. Process for the preparation of amides of formula (III) :

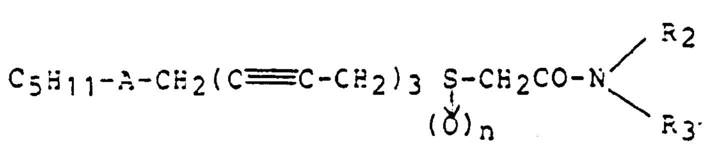

(III)

in which A, n, R$_2$ and R$_3$ have the meanings shown in Claim 1, characterized in that an activated form of the compound of formula (I'):

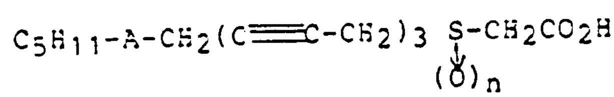

(I')

is reacted in a solvent medium with an amine of formula:

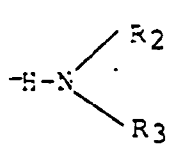

in which R$_2$ and R$_3$ have the meaning shown above.

9. Process for the preparation of amides corresponding to the formula (III):

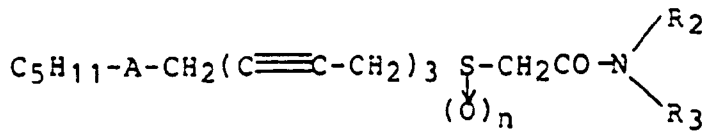 (III)

in which A, n, $R_2$ and $R_3$ have the meanings shown in Claim 1, characterized in that the 1-halo-2,5,8-heptadecatriyne of formula (4):

$C_8H_{17}$-C≡C-$CH_2$-C≡C-$CH_2$-C≡$CH_2$X (4)

or else the 1-halo-2,5,8,11-heptadecatetrayne of formula (11):

$C_5H_{11}$(C≡C-$CH_2$)$_4$-X (11)

is treated with the thiolate prepared by the action of an amine of formula:

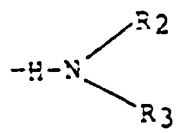

in which $R_2$ and $R_3$ have the meanings shown in Claim 1 on ethyl thioglycolate.

**10.** Process for the preparation of the sulphoxides and the sulphones corresponding to the general formula (I), in which n is equal to 1 or 2, characterized in that the thioethers prepared according to any one of the processes defined in any one of Claims 5 to 9 are reacted with 1 or 2 equivalents of an organic peracid in an organic solvent.

**11.** Medication characterized in that it contains a compound such as defined in any one of Claims 1 to 4.

**12.** Pharmaceutical composition intended to be administered by a systemic route or by a local route, characterized in that it contains at least one compound such as defined in any one of Claims 1 to 4, in the presence of a pharmaceutically acceptable carrier or diluent.

**13.** Pharmaceutical composition intended to be administered topically, presented in the form of cream, of tincture, of ointment, of pomade, of powder, of patch, of saturated pad, of solution, of gel, of lotion, of spray, of shampoo, of suspension, of microdispersion of ionic or nonionic, lipidic microcapsules, characterized in that it contains at least one compound such as defined in any one of Claims 1 to 4.

**14.** Pharmaceutical composition intended to be administered by an ocular route, presented in the form of eye lotion or pomade, characterized in that it contains at least one compound such as defined in one of Claims 1 to 4.

**15.** Pharmaceutical composition intended to be administered by air passage route which is in the form of sprays, of solutions or of suspensions, characterized in that it contains at least one compound such as defined in one of Claims 1 to 4.

**16.** Pharmaceutical composition presented in the form of a composition intended to be administered parenterally by an intravenous, intraperitoneal, intramuscular, subcutaneous or intradermal route, characterized in that it contains a compound such as defined in any one of Claims 1 to 4, in a pharmaceutically acceptable diluent.

**17.** Pharmaceutical composition intended to be administered enterally, presented in the form of tablet, of gelatin capsule, of pill, of suppository, of syrup, of suspension, of dispersion, of solution, of powder, of granule or of emulsion, characterized in that it contains at least one compound such as defined in any one of Claims 1 to 4.

**18.** Pharmaceutical composition according to any one of Claims 12 to 17, characterized in that the active compound of formula (I) is present in proportions of between 0.01 and 10% by weight relative to the total weight of the composition, and preferaly between 0.1 and 5% by weight.

**19.** Pharmaceutical composition according to Claim 12 or 13, characterized in that the compound of formula

(I) is used in combination with a hydrating, antiseborrhoeic, antibiotic, antiinflammatory or antipsoriatic agent, an agent promoting the fresh growth of hair, a carotenoid, an inhibitor of $A_2$ phospholipase or an antifungal agent.

**20.** Use of a compound such as defined in any one of Claims 1 to 4 for the preparation of a medication intended for the treatment and for the prophylaxis of allergic diseases and in the treatment of dermatitis and of inflammatory diseases such as psoriasis, eczema or acne.

**21.** Use according to Claim 20 of a compound such as defined in any one of Claims 1 to 4, for the preparation of a medication intended for the treatment and for the prophylaxis of allergic diseases, for the treatment of dermatitis and of inflammatory diseases such as psoriasis, eczema or acne, in dosages of 0.05 to 500 mg/kg/day and preferably 0.5 to 100 mg/kg/day.

**22.** Cosmetic composition characterized in that it contains at least one compound such as defined in any one of Claims 1 to 4, in proportions between 0.01 and 10% by weight, and preferably of between 0.1 and 5% by weight relative to the total weight of the composition, in the presence of a cosmetically acceptable carrier.

**23.** Application of a compound such as defined in any one of Claims 1 to 4, as a cosmetic product.